(19) Europäisches Patentamt
European Patent Office
Office européen des brevets

(11) EP 4 316 491 A1

(12) **EUROPEAN PATENT APPLICATION**
published in accordance with Art. 153(4) EPC

(43) Date of publication:
**07.02.2024 Bulletin 2024/06**

(21) Application number: **22779109.2**

(22) Date of filing: **31.03.2022**

(51) International Patent Classification (IPC):
***A61K 31/519*** *(2006.01)* ***A61P 35/00*** *(2006.01)*

(52) Cooperative Patent Classification (CPC):
**A61K 31/517; A61K 31/519; A61K 31/5377; A61P 35/00; A61P 35/04**

(86) International application number:
**PCT/CN2022/084650**

(87) International publication number:
**WO 2022/206929 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.04.2021 CN 202110358192**

(71) Applicant: **Shanghai Pharmaceuticals Holding Co., Ltd.**
**Shanghai 201203 (CN)**

(72) Inventors:
- **LI, Di**
  **Shanghai 201203 (CN)**
- **DUAN, Lingjun**
  **Shanghai 201203 (CN)**
- **HONG, Yuan**
  **Shanghai 201203 (CN)**
- **ZHANG, Zhihui**
  **Shanghai 201203 (CN)**
- **XIA, Guangxin**
  **Shanghai 201203 (CN)**
- **KE, Ying**
  **Shanghai 201203 (CN)**

(74) Representative: **Ipside**
**6, Impasse Michel Labrousse**
**31100 Toulouse (FR)**

# (54) APPLICATION OF COMPOUND IN PREPARATION OF INHIBITORY DRUG TARGETING ERBB2 MUTANT

(57) An application of a compound having a structure represented by chemical formula 1, a pharmaceutically acceptable salt thereof, a solvate thereof, a solvate of the pharmaceutically acceptable salt thereof, or a crystal form thereof, in particular an application thereof in the preparation of an inhibitory drug targeting an ErbB2 mutant. The provided compound has inhibitory activity against the ErbB2 mutant, and can effectively inhibit proliferation of ErbB2 mutation tumor cells.

EP 4 316 491 A1

## Description

The present application claims the priority of Chinese patent application 2021103581923 filed on April 1, 2021. The contents of the above Chinese patent application are incorporated herein by reference in its entirety.

## TECHNICAL FIELD

The present disclosure relates to the field of biomedicine, specifically to a use of a compound in the manufacture of an inhibitory medicament targeting an ErbB2 mutant.

## BACKGROUND

Amplification of the ErbB2 gene or overexpression of its protein (referred to as ErbB2-positive) is a common mechanism for activating ErbB2. Research over the past two decades has confirmed that overexpression of ErbB2 protein, either through gene amplification or transcriptional inactivation, is frequently associated with the oncogenesis of various solid tumors. In clinical practice, tumors that are ErbB2-positive include breast cancer, gastric cancer, esophageal cancer, ovarian cancer, endometrial cancer, colorectal cancer, lung cancer, etc. Higher levels of ErbB2 expression are observed particularly in cases of breast and gastric cancer. ErbB2 serves as a recognized therapeutic target in both breast and gastric cancer, owing to its role as an oncogenic driver.

As our understanding of the cancer genome atlas continues to evolve, periodic somatic mutations in ErbB2 have been identified, typically occurring in the absence of gene amplification. Unlike other mutated oncogenes such as BRAF or KRAS, there is no single dominant mutated allele in ErbB2. The precise distribution of mutations varies depending on the type of tumor (Nature, 2018, 554(7691): 189-194). ErbB2 mutations occur at a low frequency in several types of tumors, especially in breast cancer (3%), colon cancer (2 to 3%), and lung cancer (2 to 3%). ErbB2 mutations have also been reported in other types of human tumors, including head and neck cancer, bladder cancer, gastric cancer, ovarian cancer, and liver cancer (Oncotarget, 2018, 9(11): 9741-9750). Additionally, reports indicate that some patients who are ErbB2-positive may develop resistance to anti-ErbB2 therapies.

Various mechanisms of resistance suggest that mutations in ErbB2 represent a key mechanism. Activation of ErbB2 mutations can be triggered by three types of somatic molecular alterations: insertions and missense mutations in the kinase domain, missense mutations in the extracellular domain, or deletion mutations in the extracellular domain, resulting in truncated forms of ErbB2 (J Clin Oncol, 2020). Perhaps partly due to the absence of effective targeted therapies, the prognosis for ErbB2 mutations is less favorable compared to that of other oncogenic drivers.

Lung cancer, one of the most frequently diagnosed cancers, features ErbB2 mutations as an oncogenic driver with an incidence of 2 to 3% in non-small cell lung cancer (NSCLC) and up to 6.7% in EGFR/ALK/ROS1 triple-negative NSCLC (BMC Cancer 2016; 16: 828). While ErbB2 mutations are relatively rare in lung adenocarcinoma, given the substantial prevalence of this cancer type, these mutations still hold potential clinical significance. According to published literature, lung cancer patients with ErbB2 mutations exhibit a higher incidence of brain metastases during treatment compared to those with KRAS mutations (28% vs. 8%). There is also a trend of higher incidence compared to those with EGFR mutations (28% vs. 16%) (Cancer, 2019 Aug 30.).

CN109422755A discloses a class of compounds capable of effectively inhibiting wildtype ErbB2. However, due to structural alterations caused by ErbB2 mutations, ErbB2 mutants develop resistance to ErbB2 inhibitors. Consequently, there is an urgent need for effective inhibitors targeting ErbB2 mutants.

## CONTENT OF THE PRESENT INVENTION

The technical problem to be solved in the present disclosure is to overcome the limitations of the prior art in lacking effective inhibitors targeting an ErbB2 mutant, and to provide a use of a compound in the manufacture of an inhibitory medicament targeting the ErbB2 mutant.

One of the technical solutions provided in the present disclosure is: a use of a compound having a structure of chemical formula 1, a pharmaceutically acceptable salt thereof, a solvate thereof, a solvate of the pharmaceutically acceptable salt thereof, or a crystal form thereof in the manufacture of an inhibitory medicament targeting the ErbB2 mutant;

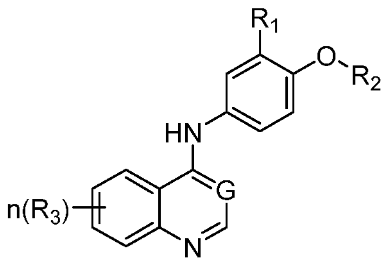
;

Chemical formula 1

wherein $R_1$ is halogen or $C_{1-}C_6$ alkyl;
$R_2$ is

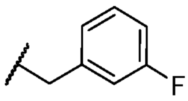, 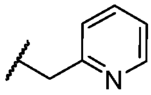, 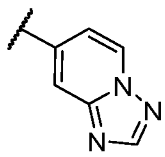, 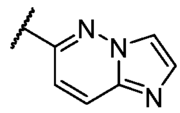, 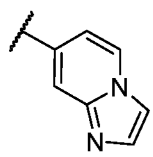,

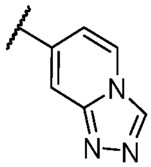, 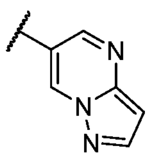, 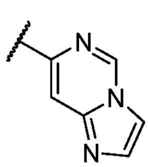, or 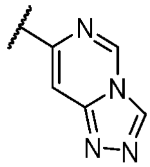;

each $R_3$ is independently

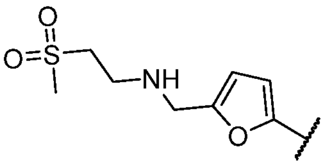, 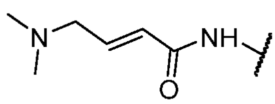, 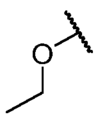,

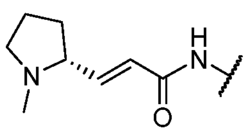, 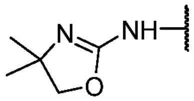, 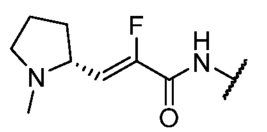, 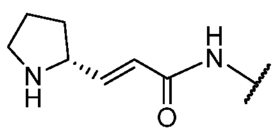,

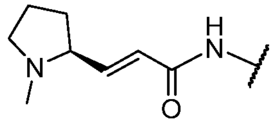, 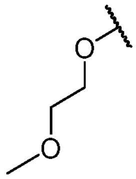, 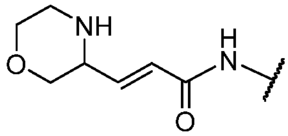, or 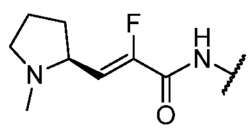;

n is 1 or 2;
G is N or C-CN.

In one embodiment, in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_{1-}C_6$ alkyl;
$R_2$ is

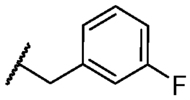, 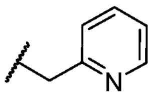, or 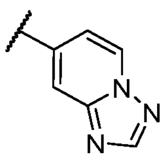;

each $R_3$ is independently

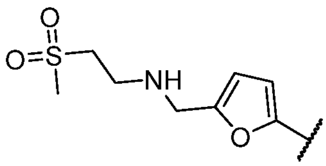, 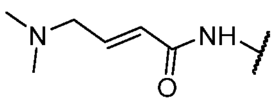, 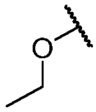,

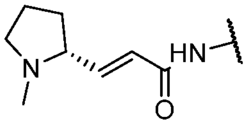, 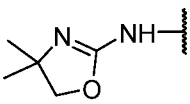, or 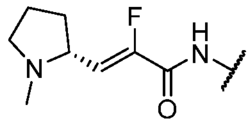;

n is 1 or 2;
G is N or C-CN.

In one embodiment, in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_{1-}C_6$ alkyl;
$R_2$ is

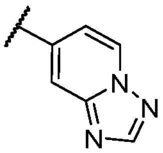, 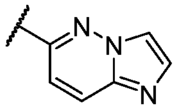, 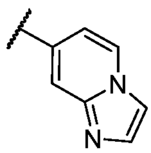, 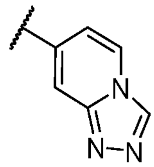, 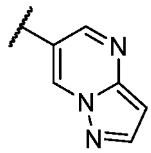,

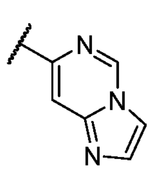, or 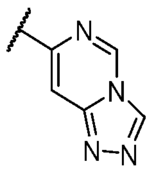;

each $R_3$ is independently

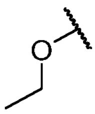, 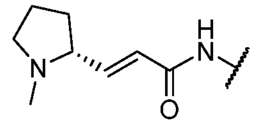, 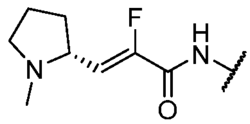,

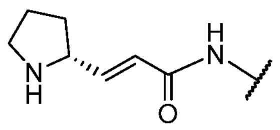, 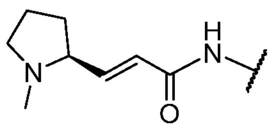, 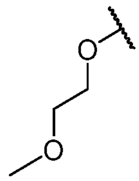, 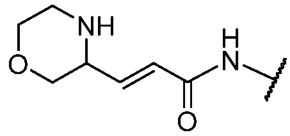,

or

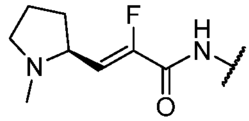;

n is 1 or 2;
G is N or C-CN.

One of the technical solutions provided in the present disclosure is: a use of a compound having a structure of chemical formula 1, a pharmaceutically acceptable salt thereof, a solvate thereof, a solvate of the pharmaceutically acceptable salt thereof, or a crystal form thereof in the manufacture of a medicament for the treatment or prevention of a disease related to an ErbB2 mutant;

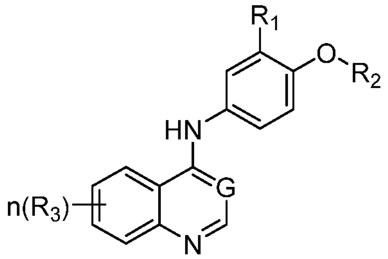;

Chemical formula 1

wherein $R_1$ is halogen or $C_{1-}C_6$ alkyl;

$R_2$ is

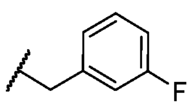, 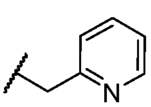, 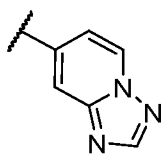, 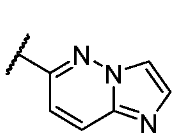, 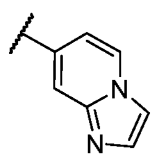,

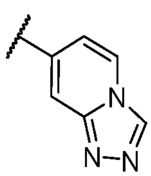, 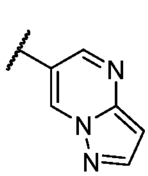, 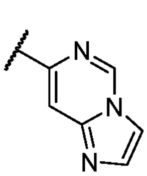, or 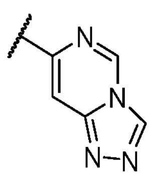;

each $R_3$ is independently

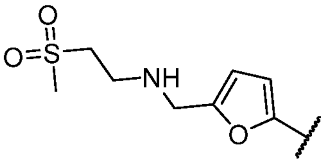, 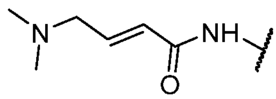, 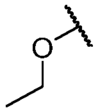,

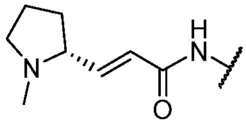, 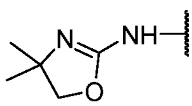, 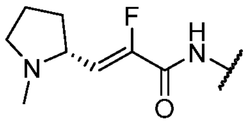, 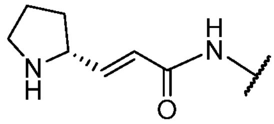,

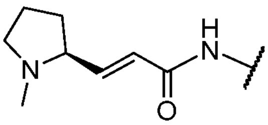, 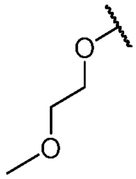, 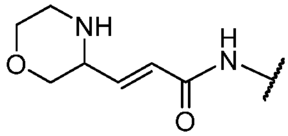, or 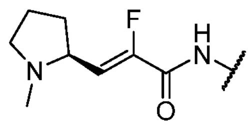;

n is 1 or 2;

G is N or C-CN.

In one embodiment, in the compound having a structure of chemical formula 1, the pharmaceutically acceptable salt thereof, the solvate thereof, the solvate of the pharmaceutically acceptable salt thereof, or the crystal form thereof;

$R_1$ is halogen or $C_1$-$C_6$ alkyl;
$R_2$ is

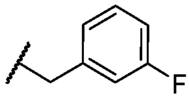, 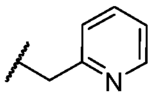, or 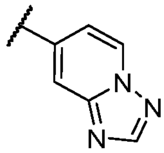;

each $R_3$ is independently

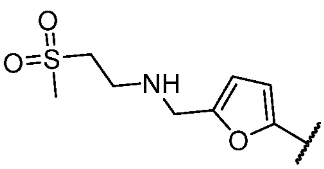, 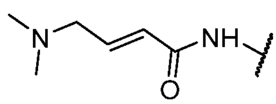, 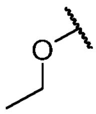,

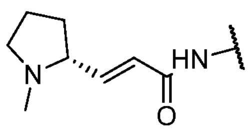, 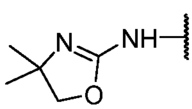, or 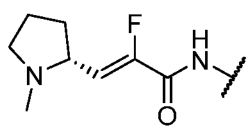;

n is 1 or 2;
G is N or C-CN.

In one embodiment, in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_1$-$C_6$ alkyl;
$R_2$ is

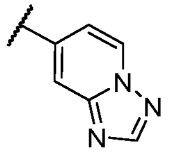, 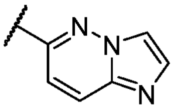, 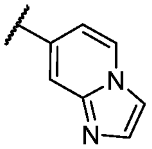, 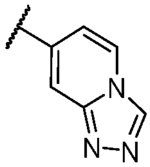, 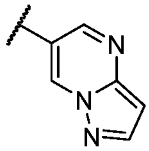,

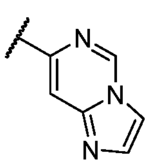, or 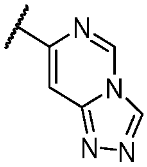;

each $R_3$ is independently

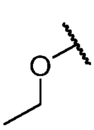, 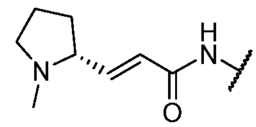, 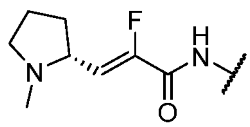,

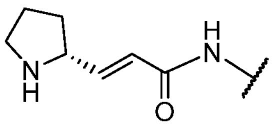, 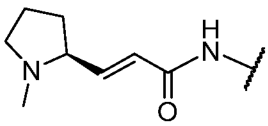, 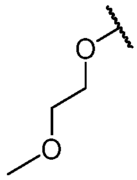, 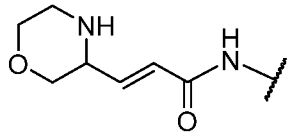,

or

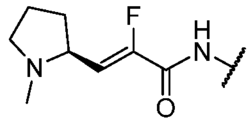;

n is 1 or 2;
G is N or C-CN.

In the chemical formula 1 of the present disclosure, when $R_1$ is halogen, the halogen is preferably Cl; when $R_1$ is $C_1$-$C_6$ alkyl, the $C_1$-$C_6$ alkyl is preferably methyl.

In one embodiment of the present disclosure, $R_2$ is

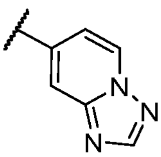, 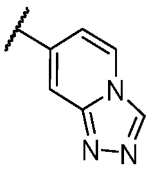,

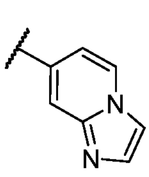, 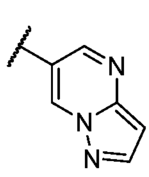, 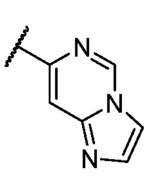, or 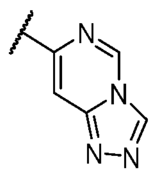.

Additionally, in one embodiment of the present disclosure,

when n is 1, $R_3$ is preferably

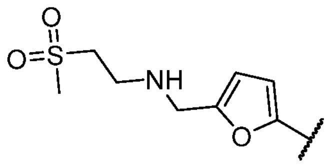, 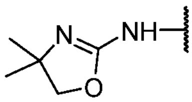,

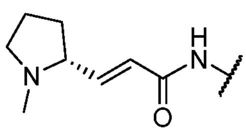, 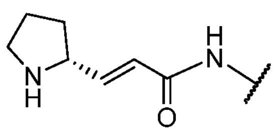, 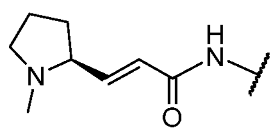, 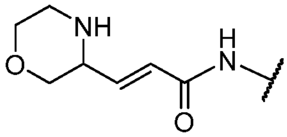, or

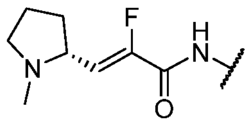,

more preferably

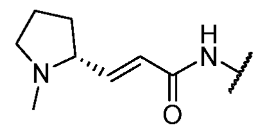, 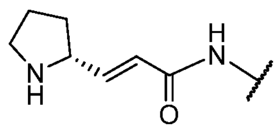,

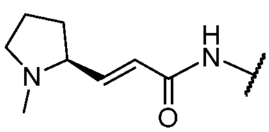, 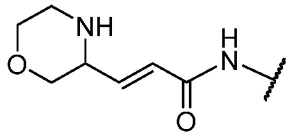, or 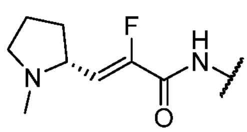;

alternatively, when n is 2, $R_3$ is preferably two neighboring groups, one of which is

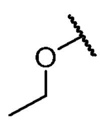 or 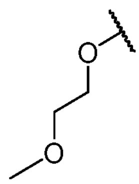,

and the other group is

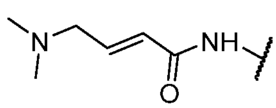, 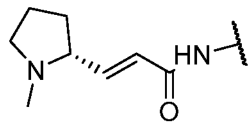,

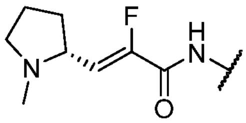, or 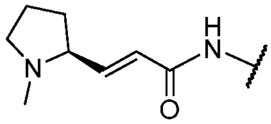,

preferably

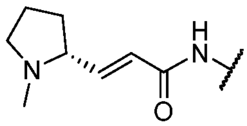,

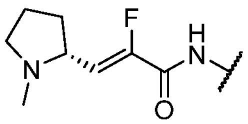, or 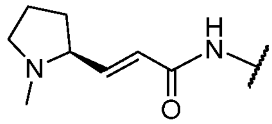.

The compound having a structure of chemical formula 1 is preferably one or more compounds selected from (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide, Lapatinib, Tucatinib, Pyrotinib, Neratinib,

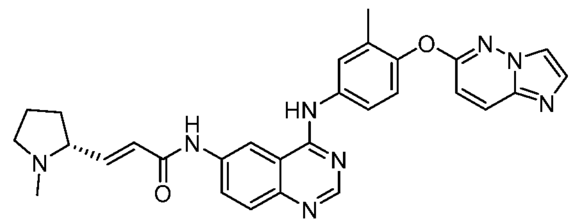,

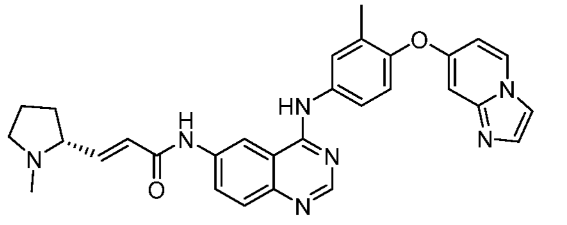, 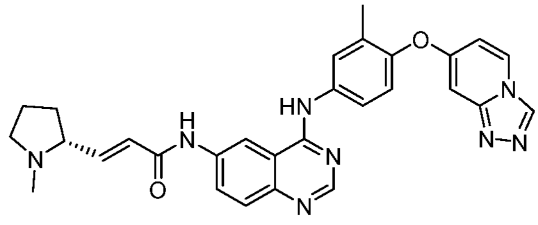,

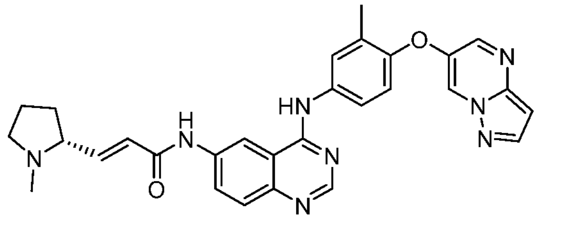, 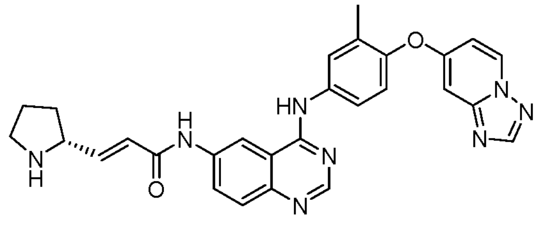,

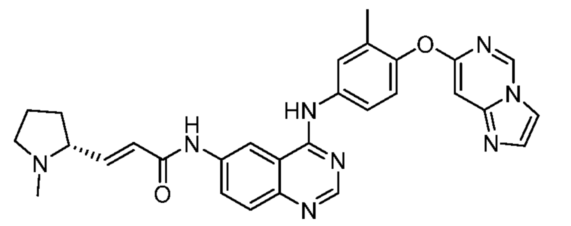,

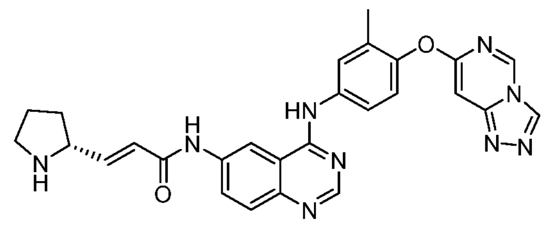,

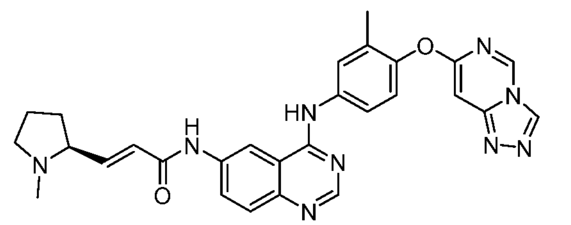,

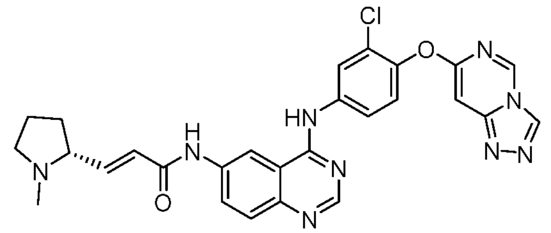,

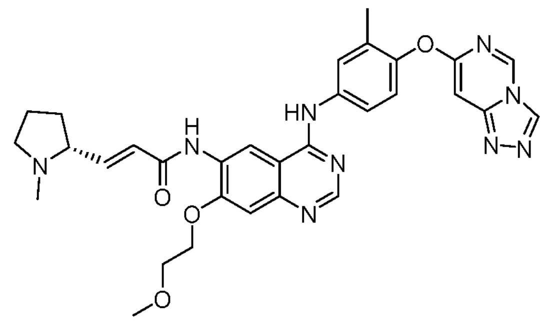,

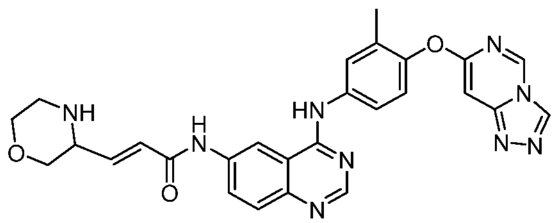,

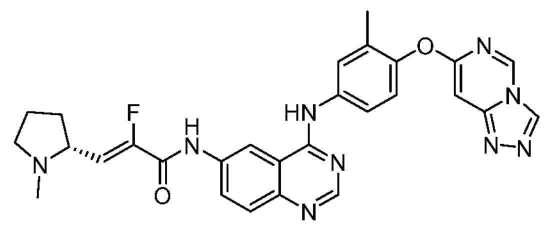,

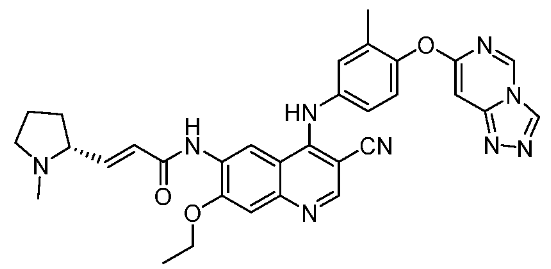,

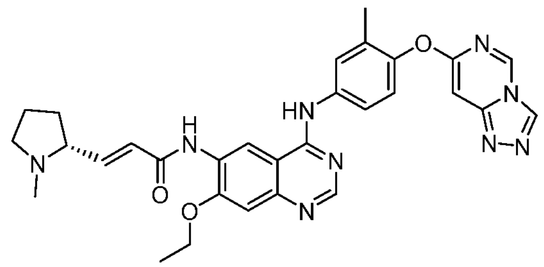,

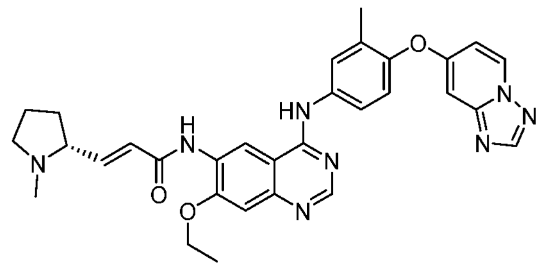,

and

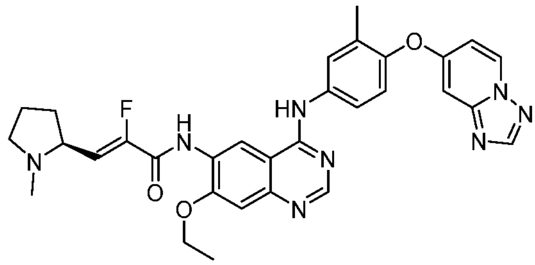

.

In the present disclosure, the ErbB2 mutant has a mutation type preferably including one or more of D769H, D769Y, R896C, V777L, G766>VC, and A775_G776insYVMA.

In one preferred embodiment of the present disclosure, the ErbB2 mutation type is D769H, D769Y, R896C, V777L, G766>VC, or A775_G776insYVMA, and the compound having a structure of chemical formula 1 is (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide.

In another preferred embodiment of the present disclosure, the ErbB2 mutation type is D769H, D769Y, R896C, V777L, or A775_G776insYVMA, and the compound having a structure of chemical formula 1 is Lapatinib.

In another preferred embodiment of the present disclosure, the ErbB2 mutation type is D769H, D769Y, R896C, or V777L, and the compound having a structure of chemical formula 1 is Tucatinib.

In another preferred embodiment of the present disclosure, the ErbB2 mutation type is D769H, D769Y, R896C, or V777L, and the compound having a structure of chemical formula 1 is Pyrotinib.

In another preferred embodiment of the present disclosure, the ErbB2 mutation type is D769H, D769Y, R896C, or V777L, and the compound having a structure of chemical formula 1 is Neratinib.

In one preferred embodiment of the present disclosure, the rbB2 mutation type is D769H, D769Y, R896C, or V777L, and the compound having a structure of chemical formula 1 is any one of the following structures:

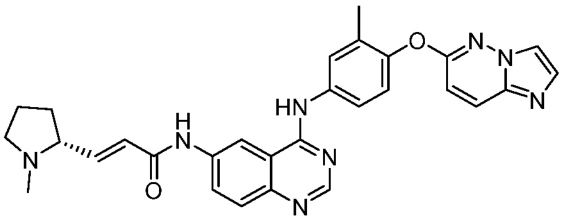

,

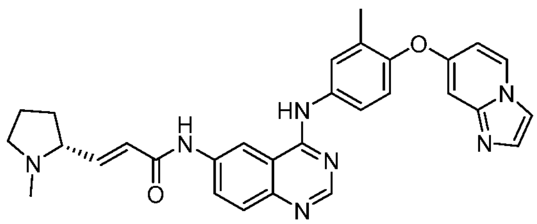

,

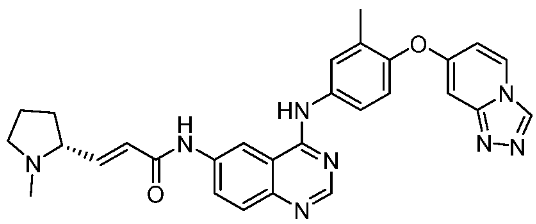

,

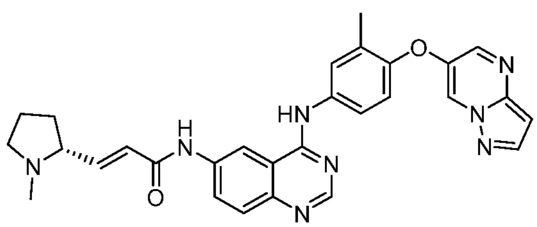

,

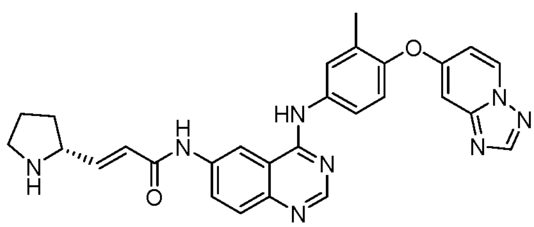

,

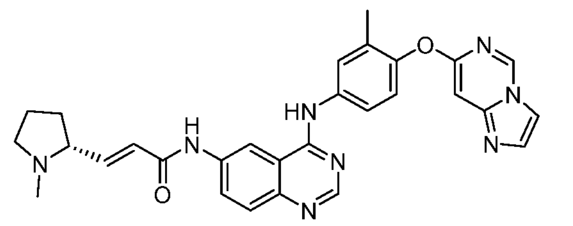
,
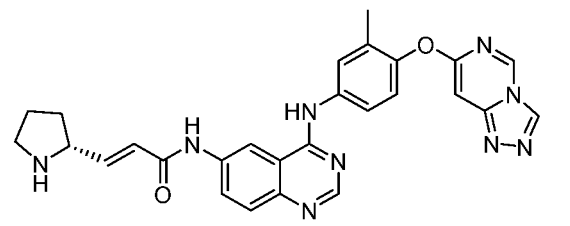
,
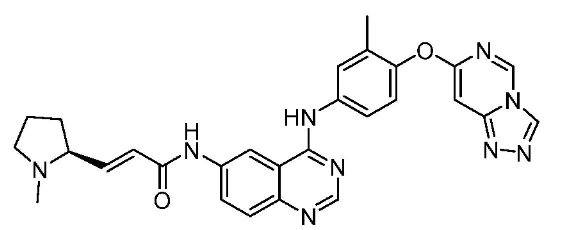
,
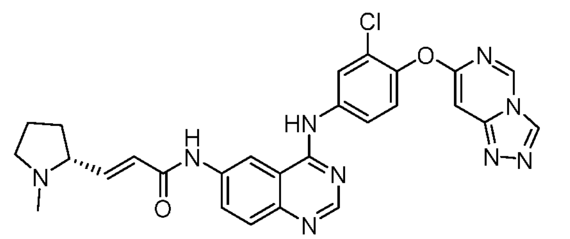
,
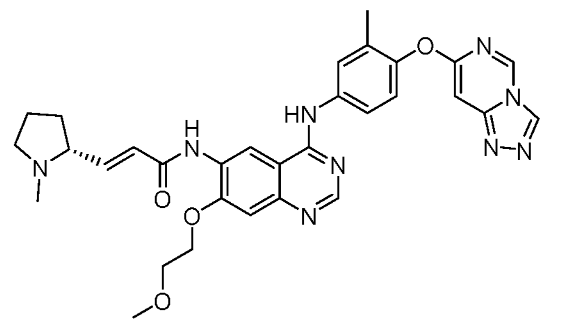
,
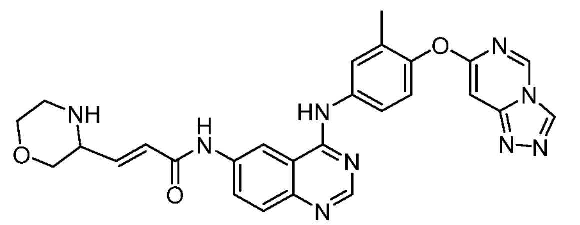
,
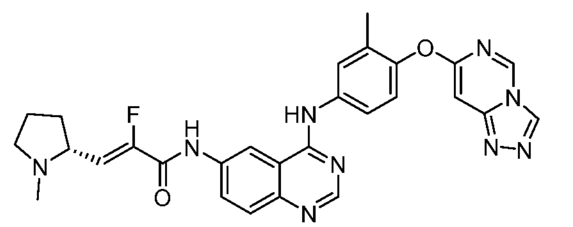
,
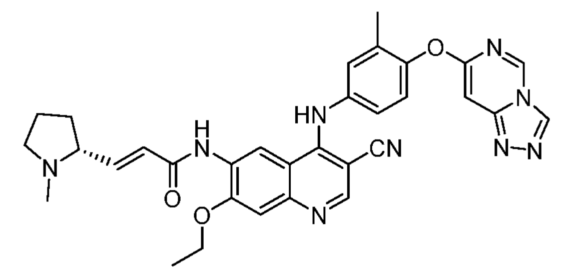
,
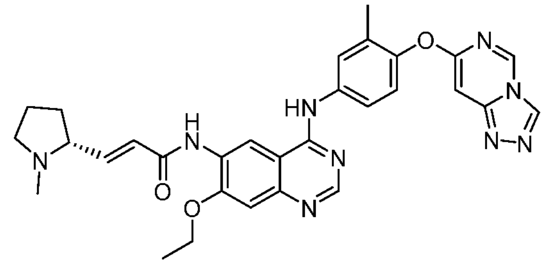
,
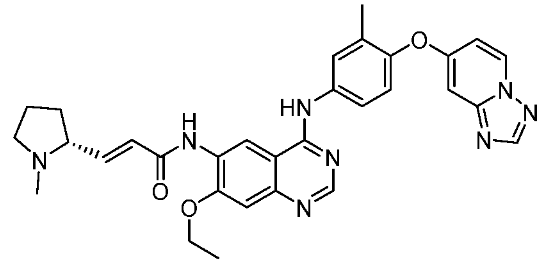
,

or

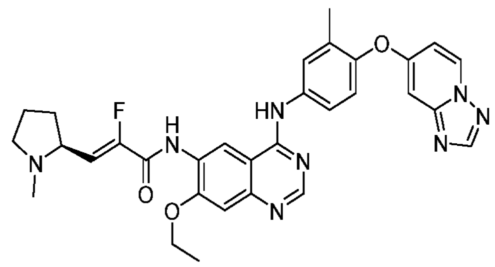

;

preferably

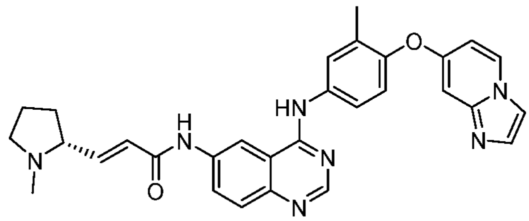

,

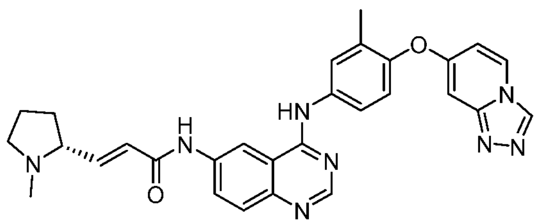

,

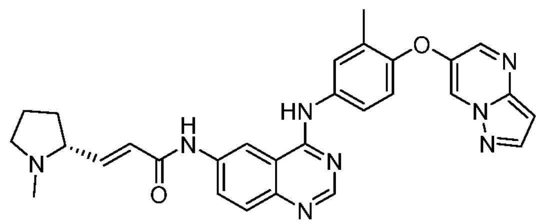

,

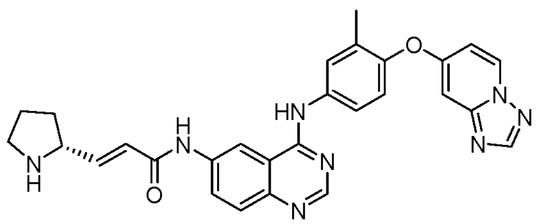

,

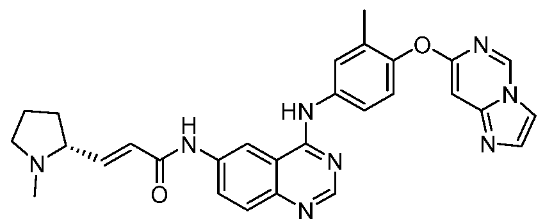

,

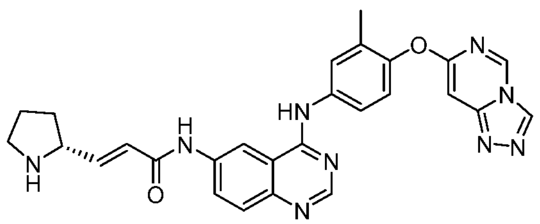

,

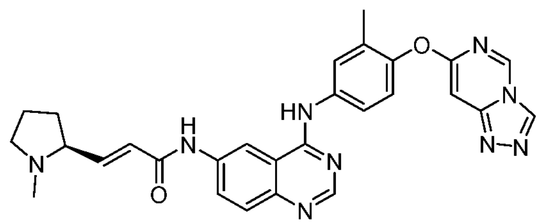

,

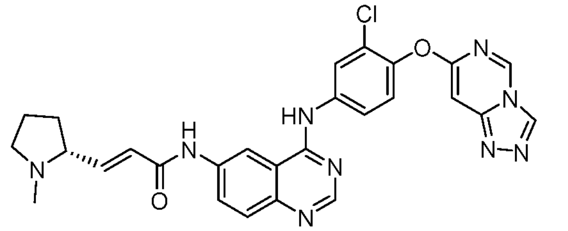
,
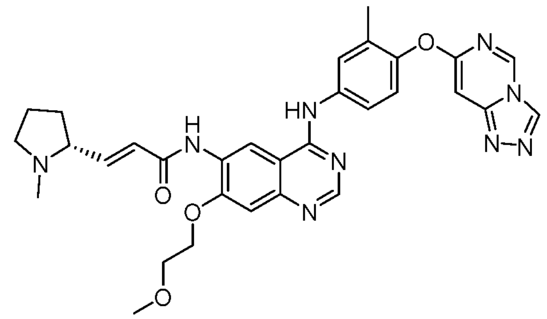
,
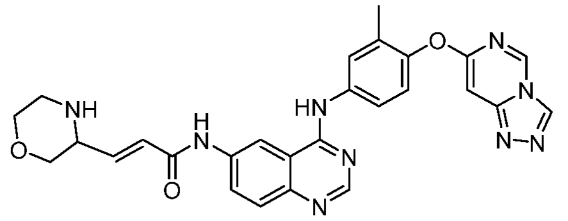
,
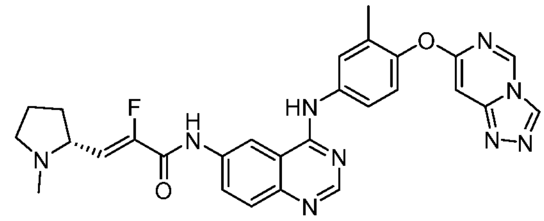
,
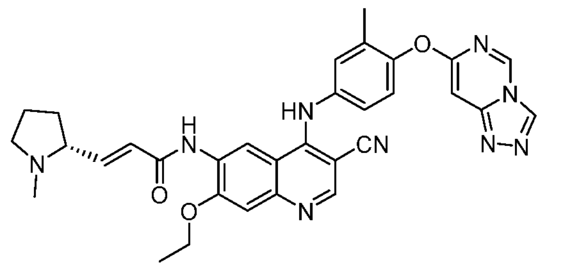
,
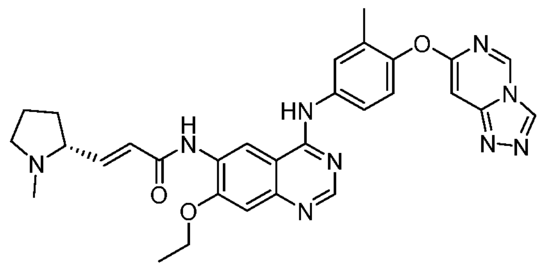
,
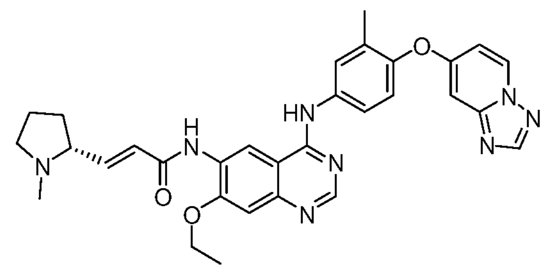
, or
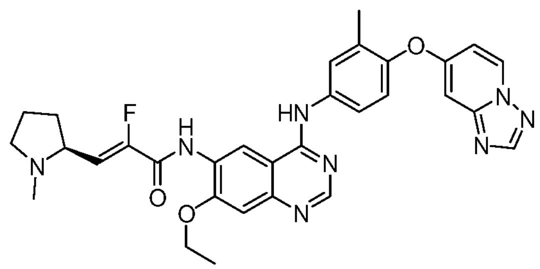
.

In the present disclosure, the disease related to ErbB2 mutations is preferably ErbB2 mutant cancer;

the ErbB2 mutant cancer preferably includes breast cancer, colon cancer, head and neck cancer, bladder cancer, gastric cancer, ovarian cancer, liver cancer, or lung cancer;
the lung cancer is further preferably non-small cell lung cancer.

In the present disclosure, the treatment is preferably inhibition of tumor metastasis and/or growth, wherein the inhibition of tumor growth includes suppressing the growth of primary tumors and metastasized tumors, wherein the metastasized tumors are preferably brain metastases.
In the present disclosure, the medicament preferably further comprises a pharmaceutically acceptable carrier.
In the present disclosure, the compound having a structure of chemical formula 1 is preferably the sole active ingredient of the medicament, or act as an active ingredient in conjunction with other ErbB2 mutant inhibitors or medicaments for the treatment of cancer.
In the present disclosure, the content of the compound having a structure of chemical formula 1 is preferably the content that achieves a therapeutically effective amount.
In the present disclosure, the compound (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide is represented as compound I:

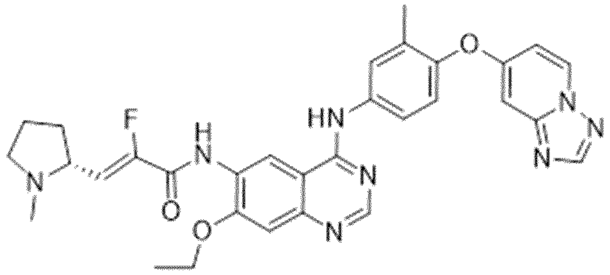

Compound I.

As used herein, the term "halogen" refers to elements in Group VIIA of the periodic table.

As used herein, the term "alkane" refers to a compound in which all carbon atoms in the molecule are connected by single carbon-carbon bonds, and the remaining valence bonds are formed with hydrogen.

As used herein, the term "treatment" can be used to denote both therapeutic and prophylactic treatment. Herein, prevention can be used to indicate that an individual's pathological condition or disease is alleviated or mitigated. The term "treatment" includes a use for the treatment of diseases in mammals, including humans, or any form of administration. Additionally, the term includes inhibiting or slowing down a disease or the progression of a disease. It encompasses the notion of restoring or repairing impaired or lost functions, thereby partially or fully alleviating the disease; stimulating inefficient processes; or alleviating severe conditions.

As used herein, the term "therapeutically effective amount" or "pharmaceutically effective amount" refers to an amount of a compound or composition sufficient to prevent or treat the disease under discussion. It is an amount that is sufficient to treat the disease with a reasonable benefit-to-risk ratio and is medically appropriate without causing adverse effects. The level of an effective amount can be determined based on factors including the patient's health condition, severity of the disease, activity of the drug, patient's sensitivity to the drug, mode of administration, time of administration, route of administration and excretion rate, duration of treatment, preparation or co-administered drug, and other factors well-known in the art within the medical field. In one preferred embodiment of the present disclosure, the therapeutically effective amount of compound I is 10 to 40 mg/kg, such as 10, 15, 20, 25, 30, 35, or 40 mg/kg.

Herein, the pharmaceutically acceptable carrier may be any carrier, as long as the carrier is a non-toxic substance suitable for delivery to the patient. It may include distilled water, alcohol, fats, waxes, and inert solids as carriers, as well as pharmaceutically acceptable adjuvants such as buffers and dispersants.

Specifically, by including the pharmaceutically acceptable carrier in addition to the active ingredient, the pharmaceutical composition can be formulated into a preparation according to its route of administration using conventional methods known in the art. As used herein, the term "pharmaceutically acceptable" means that the carrier's toxicity does not exceed the amount that the subject being administered (prescribed) can tolerate without inhibiting the activity of the active ingredient.

In accordance with common knowledge in the art, the above various preferred conditions can be arbitrarily combined to obtain various preferred examples of the present disclosure.

The reagents and raw materials used in the present disclosure are commercially available.

The positive advancement of the present disclosure lies in the fact that the compound provided by the present disclosure has inhibitory activity against the ErbB2 mutant, and can effectively inhibit the proliferation of ErbB2 mutant tumor cells. In a preferred embodiment, the compound provided by the present disclosure, particularly compound I, exhibits good inhibitory activity against the proliferation of ErbB2 mutations in NCI-H1781 and Ba/F3 cell lines. Furthermore, in a mouse xenograft model using the B-cell lymphoma cell line Ba/F3 ErbB2 A775_G776insYVMA, it can effectively inhibit tumor growth. This demonstrates the potential clinical application value of such compounds.

## DETAILED DESCRIPTION OF THE PREFERRED EMBODIMENT

The present disclosure is further described below by the way of examples, but is not thereby limited to the scope of the described examples. In the following examples, experimental methods for which specified conditions are not specified are selected according to conventional methods and conditions, or according to the product instructions.

### Example 1: Preparation of compound I

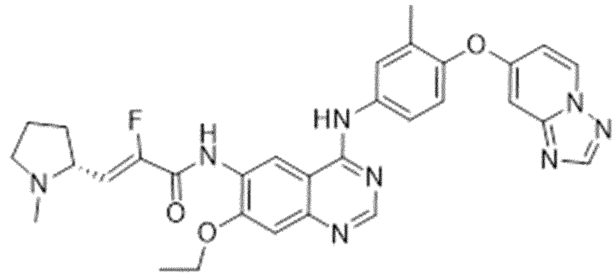

Compound I

Preparation of compound I: [(R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide]

Step A: Preparation of 7-ethoxy-6-nitroquinazolin-4-ol: 7-Fluoro-6-nitroquinazolin-4-ol (4000 mg, 19.13 mmol) was dissolved in tetrahydrofuran (20 mL). In an ice-water bath, the mixture was cooled to 0°C, and to the reaction mixture was slowly dropwise added a solution of sodium ethoxide (4000 mg, 58.78 mmol) in anhydrous ethanol (20 mL). The mixture was gradually warmed to room temperature and stirred for 16 hours. In an ice-water bath, the reaction mixture was added with acetic acid to adjust the pH to 5 to 6, then filtered, and dried under vacuum to obtain 4000 mg of a light yellow solid, which was directly used in the next reaction step.

Step B: Preparation of 4-chloro-7-ethoxy-6-nitroquinazoline: 7-Ethoxy-6-nitroquinazolin-4-ol (4000 mg, 17.01 mmol) was dissolved in phosphorus oxychloride (50 mL), and the mixture was heated under reflux with stirring for 4 hours. Subsequently, the reaction mixture was concentrated under reduced pressure, and the residue was dissolved in dichloromethane (500 mL). The mixture was sequentially washed with water (500 mL) and saturated brine (500 mL), dried over anhydrous sodium sulfate for 2 hours, and concentrated under reduced pressure to obtain 4000 mg of a pale yellow solid with a yield of 92.7%, which was directly used in the next reaction step.

Step C: Preparation of N-(4-([1,2,4]triazolo[1,5-a]pyridin-7-yloxy)-3-methylphenyl)-7-ethoxy-6-nitroquinazolin-4-amine: 4-([1,2,4]Triazolo[1,5-a]pyridin-7-yloxy)-3-methylaniline (250 mg, 1.04 mmol), 4-chloro-7-ethoxy-6-nitroquinazoline (400 mg, 1.58 mmol), and potassium carbonate (290 mg, 2.10 mmol) were mixed in N,N-dimethylformamide (10 mL), and the mixture was stirred at room temperature. The mixture was filtered, and to the filtrate were added dichloromethane (100 mL) and water (100 mL). The organic layer was separated, sequentially washed with water (100 mL) and saturated brine (100 mL), and then dried over anhydrous sodium sulfate. The mixture was filtered, and the filtrate was concentrated under reduced pressure to obtain 590 mg of a yellow solid, which was directly used in the next reaction step.

Step D: Preparation of N4-(4-([1,2,4]triazolo[1,5-a]pyridin-7-yloxy)-3-methylphenyl)-7-ethoxyquinazolin-4,6-diamine: N-(4-([1,2,4]triazolo[1,5-a]pyridin-7-yloxy)-3-methylphenyl)-7-ethoxy-6-nitroquinazolin-4-amine (590 mg, 1.29 mmol) was dissolved in methanol (20 mL). Raney nickel was added thereto, and the reaction mixture was replaced with argon three times. Hydrogen was introduced, and the reaction mixture was stirred at room temperature for 2 hours. The reaction mixture was filtered through diatomite, and concentrated under reduced pressure to obtain 330 mg of a solid, which was directly used in the next reaction step.

Step E: Preparation of diethyl (2-((4-((4-([1,2,4]triazolo[1,5-a]pyridin-7-yloxy)-3-methylphenyl)amino)-7-ethoxyquinazolin-6-yl)amino)-1-fluoro-2-oxoethyl)phosphate: To pyridine (20 mL) were added 7-ethoxy-N4-[3-methyl-4-([1,2,4]triazolo[1,5-a]pyridin-7-yloxy)phenyl]quinazoline-4,6-diamine (1.15 g, 2.69 mmol) and 2-diethoxyphosphoryl-2-fluoroacetic acid (691 mg, 3.22 mmol). Phosphorus oxychloride (1.5 mL) was added dropwise thereto at 0°C, and the reaction mixture was reacted for 1.5 hours. The reaction mixture was added with sodium bicarbonate aqueous solution to quench, and concentrated under reduced pressure to obtain a residue. Dichloromethane and water were added thereto, and the organic phase was discarded. The aqueous phase was extracted with dichloromethane (60 mL × 3). The organic phases were combined, and dried over anhydrous sodium sulfate. The reaction mixture was concentrated under reduced pressure to obtain a crude product, which was purified by column chromatography to obtain 920 mg of a yellow solid with a yield of 55%.

Step F: Preparation of tert-butyl (R,Z/E)-2-(3-((4-((4-([1,2,4]triazolo[1,5-a]pyridin-7-yloxy)-3-methylphenyl)amino)-7-ethoxyquinazolin-6-yl)amino)-2-fluoro-3-oxoprop-1-en-1-yl)pyrrolidine-1-carboxylate: Sodium hydroxide (359 mg, 8.97 mmol) was dissolved in a mixed solvent of ethanol (18 mL) and water (1.8 mL), and diethyl (2-((4-((4-([1,2,4]triazolo[1,5-a]pyridin-7-yloxy)-3-methylphenyl)amino)-7-ethoxyquinazolin-6-yl)amino)-1-fluoro-2-oxoethyl)phosphate (700 mg, 1.12 mmol) was added thereto. Once the mixture was clarified, tert-butyl (2R)-2-formylpyrrolidine-1-carboxylate (447 mg, 2.24 mmol) was added thereto at 0°C. The reaction mixture was warmed to room temperature and stirred for

3 hours. The reaction mixture was added with ammonium chloride aqueous solution, and concentrated under reduced pressure to remove the organic solvent. The residue was extracted with dichloromethane (60 mL × 3). The organic phases were combined, and dried over anhydrous sodium sulfate. The reaction mixture was concentrated under reduced pressure to obtain a crude product, which was purified by column chromatography to obtain 565 mg of a yellow solid mixture with a yield of 75%.

Step G: Preparation of (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide: A mixture of tert-butyl (R,Z/E)-2-(3-((4-((4-([1,2,4]triazolo[1,5-a]pyridin-7-yloxy)-3-methylphenyl)amino)-7-ethoxyquinazolin-6-yl)amino)-2-fluoro-3-oxoprop-1-en-1-yl)pyrrolidine-1-carboxylate (540 mg, 0.81 mmol) was dissolved in dichloromethane (16 mL). Trifluoroacetic acid (4 mL) was added thereto, and the reaction mixture was stirred at room temperature for 3 hours. The reaction mixture was concentrated under reduced pressure. The resulting residue was dissolved in methanol (20 mL), and 37% formaldehyde aqueous solution (1 mL) was added thereto. The reaction mixture was stirred at room temperature for 1.5 hours, then slowly added with sodium triacetoxyborohydride (1 g, 4.69 mmol), and stirred at room temperature for 16 hours. The reaction mixture was concentrated under reduced pressure to obtain a residue. The residue was added with dichloromethane and sodium bicarbonate aqueous solution. The organic phase was discarded, and the aqueous phase was extracted with dichloromethane (80 mL × 3). The organic phases were combined, and dried over anhydrous sodium sulfate. The reaction mixture was filtered, and concentrated under reduced pressure to obtain a residue. The residue was then purified by column chromatography to obtain a crude product, which was further purified by SFC to obtain 134 mg of the target compound with a yield of 28%.

In the following examples, compounds 2 to 7, compounds 10 to 15, and compound 17 have been disclosed in WO2019042409A1 and were prepared in accordance with the methods described in that patent application; compounds 8, 9, and 16 have been disclosed in WO2017148391A1 and were prepared in accordance with the methods described in that patent application. Lapatinib, tucatinib, pyrotinib, and neratinib were commercially purchased.

**Example 2: ErbB2 mutant inhibition assay**

The reagents and materials used in the assay, as well as the testing procedure, were provided and carried out by Reaction Biology Corporation. The specific steps were as follows:

1. Compound preparation: Both the test compounds and the control compound (staurosporine) were dissolved in DMSO.
2. Conditions for the enzyme reaction assay: The test compounds were incubated for 20 minutes, ATP concentration was set at 10 μM, and the total duration of the enzyme reaction was 2 hours.
3. Experimental steps for the enzyme reaction

The enzyme reaction buffer was 20 mM HEPES at a pH of 7.5, 10 mM $MgCl_2$, 2 mM $MnCl_2$, 1 mM EGTA, 0.01% Brij35, 0.02 mg/mL BSA, 0.1 mM $Na_3VO_4$, 2 mM DTT, and 1% DMSO.

The steps for the enzyme reaction were as follows:

(1) The substrates needed for each enzyme reaction were dissolved in freshly prepared enzyme reaction buffer, respectively;
(2) each enzyme was added to the above substrate solution and well-mixed;
(3) the test compounds were added to the enzyme-substrate mixture at their corresponding concentrations, respectively, and incubation was carried out at room temperature for 20 minutes;
(4) the enzyme reaction was initiated by the addition of $^{33}$P-ATP (a final concentration of 0.01 μCi/μL), respectively, and incubation was carried out at room temperature for 2 hours;
(5) the enzyme reaction mixture was applied onto P81 ion exchange paper (Whatman # 3698-915);
(6) the paper was washed with 0.75% phosphoric acid;
(7) radiolabeled phosphorylated substrates on the paper were read.

4. Data analysis:

Kinase activity% = (Remaining kinase activity of test compound samples / Kinase activity of solvent control samples (DMSO)) * 100%

The $IC_{50}$ of the compound was calculated by fitting the kinase activity% at different compound concentrations.

Table 1

| Kinase / Test compound | Compound $IC_{50}$ (nM) | | | |
|---|---|---|---|---|
| | ErbB2 (D769H) | ErbB2 (D769Y) | ErbB2 (R896C) | ErbB2 (V777L) |
| Lapatinib | 26.5 | 381 | 5.85 | 102 |
| Tucatinib | 2.58 | 8.89 | 2.17 | 1.08 |
| Compound I | 0.716 | 1.40 | 0.713 | 0.312 |
| Pyrotinib | 15.6 | 91.7 | 5.24 | 10.5 |
| Neratinib | 15.4 | 81.7 | 4.02 | 8.88 |
| 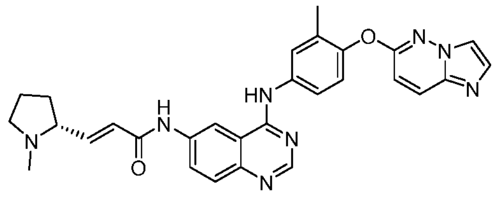 Compound 2 | 9.11 | 38.9 | 3.70 | 14.9 |
| 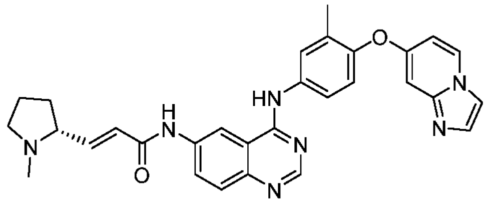 Compound 3 | <0.152 | 0.244 | 0.379 | <0.152 |
| 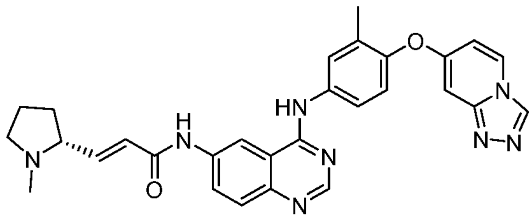 Compound 4 | 0.253 | 1.98 | 0.191 | 0.625 |
| 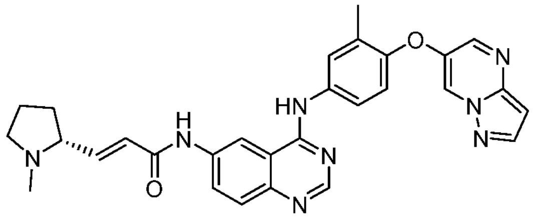 Compound 5 | <0.152 | 0.60 | 0.336 | 0.279 |
| 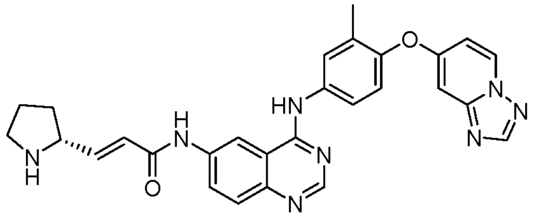 Compound 6 | 0.325 | 0.246 | 0.997 | 0.186 |

| | | | | |
|---|---|---|---|---|
| 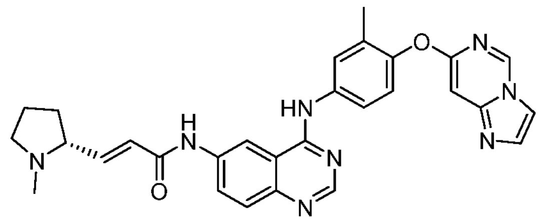 Compound 7 | 0.283 | 0.216 | 0.741 | 0.224 |
| 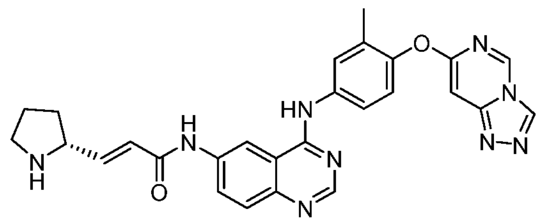 Compound 8 | 0.422 | 0.247 | 1.13 | 0.357 |
| 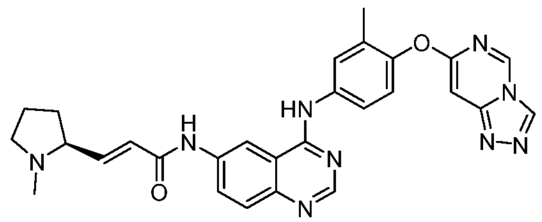 Compound 9 | 0.549 | 1.45 | 0.788 | 0.163 |
| 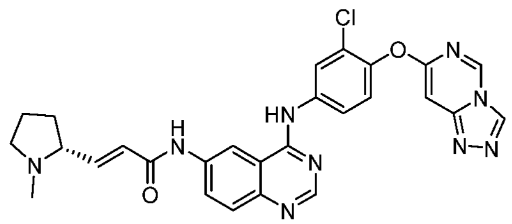 Compound 10 | <0.152 | <0.152 | 0.187 | <0.152 |
| 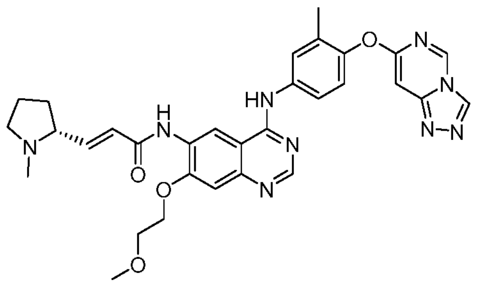 Compound 11 | 0.241 | <0.152 | 0.599 | <0.152 |
| 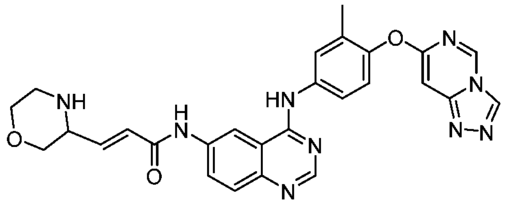 Compound 12 | 0.72 | 0.892 | 0.973 | 0.438 |

| | | | | |
|---|---|---|---|---|
| 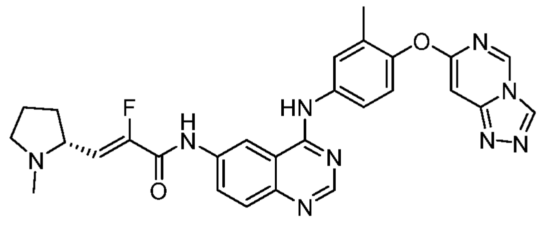 Compound 13 | 1.69 | 0.811 | 4.84 | 2.41 |
| 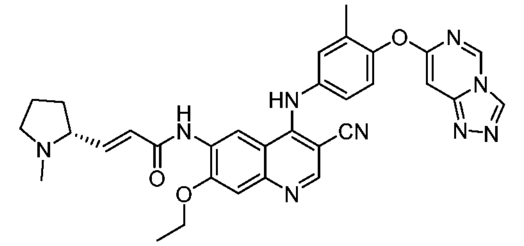 Compound 14 | 1.34 | 2.06 | 1.94 | 0.889 |
| 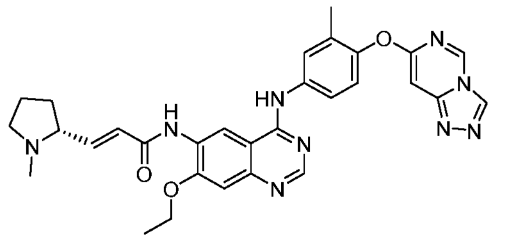 Compound 15 | 0.563 | 0.413 | 1.87 | 0.471 |
| 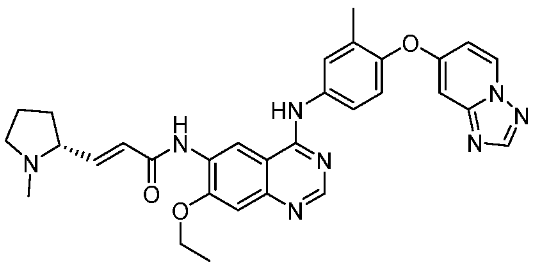 Compound 16 | <0.152 | <0.152 | <0.152 | <0.152 |
| 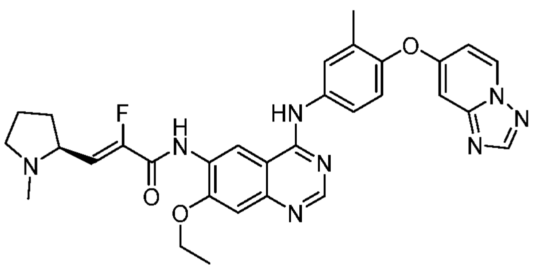 Compound 17 | 0.998 | 2.44 | 0.978 | 0.235 |
| Control compound | 64.3 | 13.8 | 51.1 | 23.0 |

The control compound used is Staurosporine, which is an effective, non-selective protein kinase inhibitor and an apoptosis inducer. Its role is to validate that the experimental system is effective.

Lapatinib, Tucatinib, Pyrotinib, Neratinib, and compound I all contain a structure of chemical formula 1, and their respective structures are as follows:

Chemical formula 1: 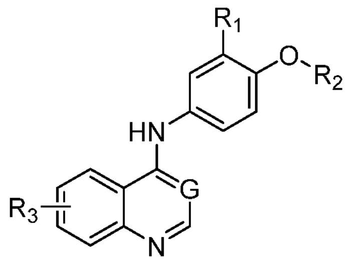

Lapatinib:

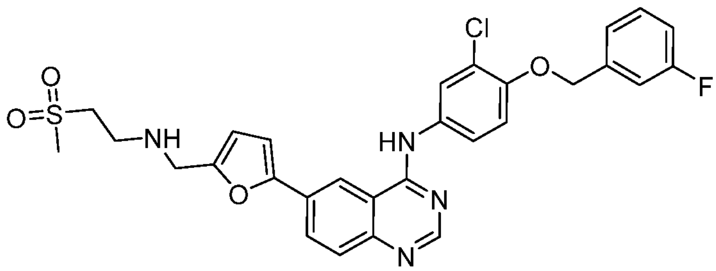

Tucatinib:

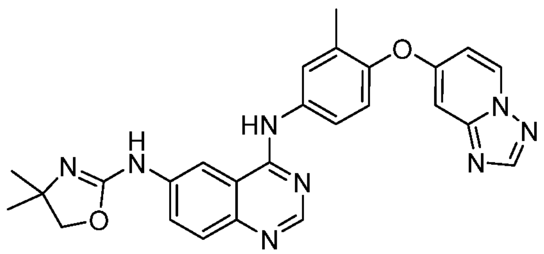

Pyrotinib:

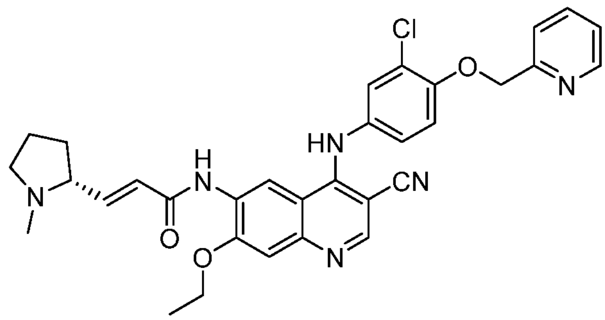

Neratinib:

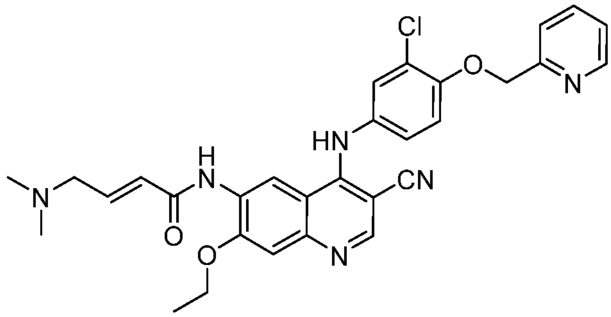

Compound I:

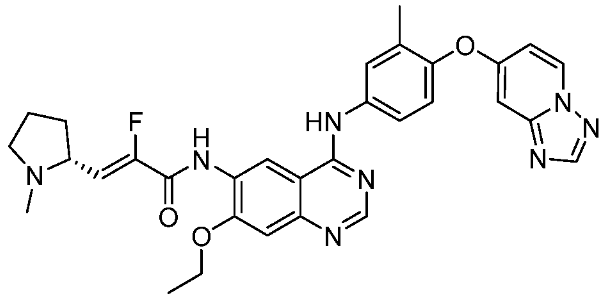

Control compound (Staurosporine):

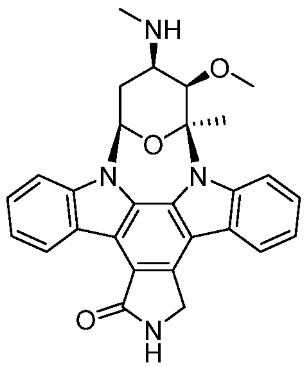

Results: Lapatinib, Tucatinib, Pyrotinib, Neratinib, and the compounds of chemical formula 1 all exhibit inhibitory potency on ErbB2 mutants. Among them, most of the compounds of chemical formula 1 have higher inhibitory potency than Lapatinib, Tucatinib, Pyrotinib, and Neratinib.

**Example 3: ErbB2 mutant cell inhibition test**

Table 2

| Cell name | Source | Cancer type | Cultivation method |
|---|---|---|---|
| Ba/F3 ErbB2 A775_G776insYVMA | KYinno Biotechnology | B-cell lymphoma | Cells were collected after culture and expansion in RPMI 1640 medium supplemented with 10% fetal bovine serum at 37°C in a 5% $CO_2$ environment. |

(continued)

| Cell name | Source | Cancer type | Cultivation method |
|---|---|---|---|
| NCI-H1781 | Crown Bioscience | Human bronchioalveolar carcinoma | Cells were collected after culture and expansion in RPMI 1640 medium supplemented with 10% fetal bovine serum at 37°C in a 5% $CO_2$ environment. |

Cells were cultured, harvested during their logarithmic growth phase, and counted using a platelet counter. Cell viability was verified to be above 90% by trypan blue exclusion assay. After *in vitro* culture, $2\times10^6$ cells were obtained. The cell suspension was aliquoted into a 96-well plate at $1\times10^3$ cells per well (90 μL/well, and incubated overnight under conditions of 37°C, 5% $CO_2$, and 95% humidity. A 10× drug solution was prepared, with a maximum concentration of 10 μM for the assay, and serially diluted 3-fold to nine concentrations. 10 μL of the drug solution was added to each well of the 96-well plate inoculated with the cells, and three duplicate wells were set for each drug concentration. The 96-well plate, now containing the drug-treated cells, was cultured under conditions of 37°C, 5% $CO_2$, and 95% humidity for an additional 72 hours before CTG analysis was performed.

Table 3

| Cell line name | Test reagents | $IC_{50}$ (μM) |
|---|---|---|
| NCI-H1781 ErbB2 exon20ins (G766>VC) | Compound I | 0.0358 |
| | Pyrotinib | 0.0732 |
| Ba/F3 ErbB2 A775_G776insYVMA | Compound I | 0.005 |
| | Lapatinib | 0.106 |

Results: Lapatinib and compound I demonstrate good inhibitory activity on cell proliferation in the aforementioned two mutant cell lines (both mutations are observed in lung cancer).

**Example 4: Efficacy testing in NPSG mouse xenograft model**

NPSG male mice (Shanghai Jihui Laboratory Animal Care Co., Ltd., Certificate: No. 20170012006783) were subcutaneously inoculated on the right shoulder with Ba/F3 ErbB2 A775_G776insYVMA cells (cultured *in vitro,* $9\times10^7$ cells obtained). When the average tumor volume reached 130 $mm^3$, the mice were randomly divided into six groups based on tumor volume and body weight, with 10 mice in each group, and administration was started immediately. After the initiation of administration, the mice's body weight and tumor volume were measured twice a week, with the administration continued until Day 13 of the experiment. On Day 14 of the experiment, post-administration sample collection was conducted for each group.

Table 4: Average tumor volume for each group of mice in the experiment

| Tumor volume ($mm^3$) | | | | | | |
|---|---|---|---|---|---|---|
| Days | Control group | Compound I 10 mg/kg | Compound I 20 mg/kg | Compound I 40 mg/kg | Pyrotinib 20 mg/kg | Neratinib 20 mg/kg |
| 0 | 130.8±5.17 | 130.9±4.72 | 130.2±5.25 | 130.1±5 | 130.4±5.4 | 130.9±4.75 |
| 4 | 235.2±12.54 | 112.1±4.71 | 69.2±2.69 | 63.8±4.44 | 154.6±4.87 | 115.3±9.81 |
| 7 | 442.8±29.94 | 157.7±6.95 | 82.7±4.74 | 36.3±11.12 | 328.2±13.39 | 182±10.87 |
| 11 | 681.1±35.37 | 160.2±6.3 | 93.5±5.75 | 21.5±9.26 | 405.4±19.38 | 325.6±9.86 |
| 13 | 1037.2±69.82 | 172.9±11.16 | 71.1±4.51 | 20.6±8.61 | 534.8±38.29 | 532.8±15.26 |
| TGI (%) | N/A | 95% | 107% | 112% | 55% | 56% |
| Note: Data are presented as mean ± standard error; control group is the group without any compound administered. | | | | | | |

Conclusion: The data indicate that in the B-cell lymphoma cell line Ba/F3 ErbB2 A775_G776insYVMA mouse xenograft model, after continuous administration for 13 days, both Pyrotinib and Lapatinib showed moderate efficacy at a dose of 20 mg/kg. Compound I exhibited strong efficacy at doses of 10 mg/kg, 20 mg/kg, and 40 mg/kg, with the 40 mg/kg dose of compound I showing the strongest tumor inhibitory effect, which was statistically different from the other test groups (*p*-value less than 0.01). No significant weight loss or mortality was observed in the animals of any test group during the experiment.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A use of a compound having a structure of chemical formula 1, a pharmaceutically acceptable salt thereof, a solvate thereof, a solvate of the pharmaceutically acceptable salt thereof, or a crystal form thereof in the manufacture of an inhibitory medicament targeting an ErbB2 mutant;

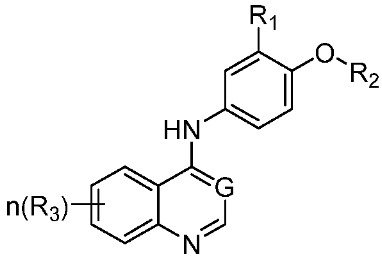 ;

Chemical formula 1

wherein $R_1$ is halogen or $C_1$-$C_6$ alkyl;
$R_2$ is

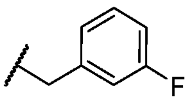 , 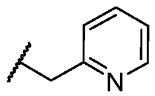 , 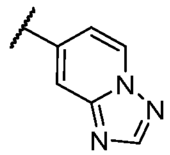 , 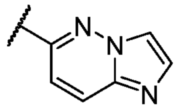 , 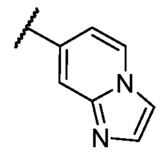 ,

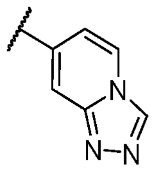 , 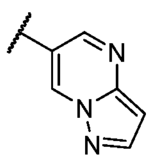 , 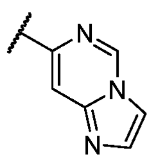 , or 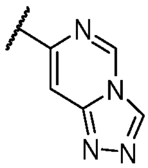 ;

each $R_3$ is independently

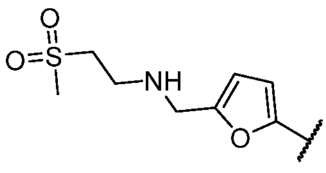 , 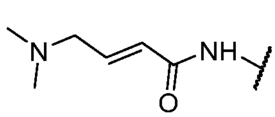 , 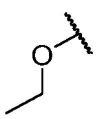 ,

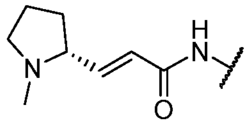, 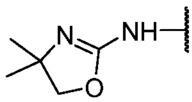, 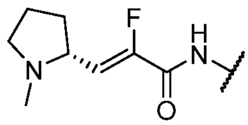, 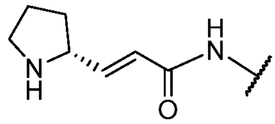,

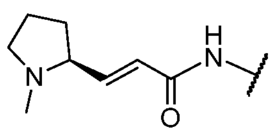, 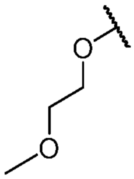, 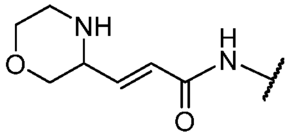, or 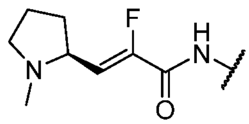;

n is 1 or 2;
G is N or C-CN.

**2.** The use according to claim 1, wherein in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_1$-$C_6$ alkyl;
$R_2$ is

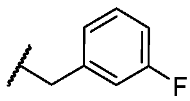, 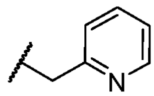, or 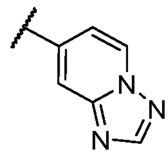;

each $R_3$ is independently

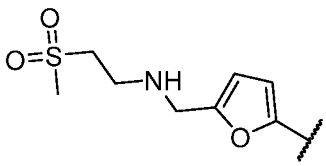, 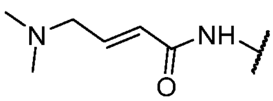, 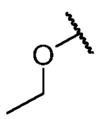,

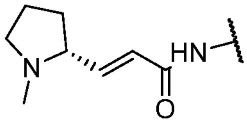, 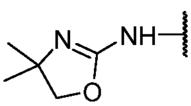, or 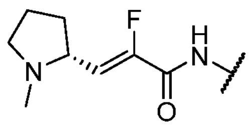;

n is 1 or 2;
G is N or C-CN.

**3.** The use according to claim 1, wherein in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_1$-$C_6$ alkyl;
$R_2$ is

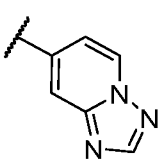 , 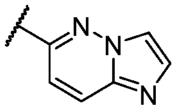 , 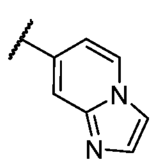 , 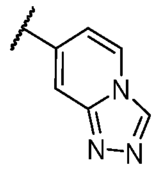 , 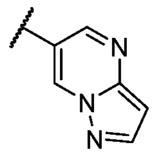 ,

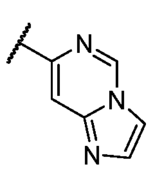 , or 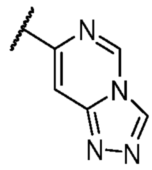 ;

each $R_3$ is independently

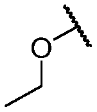 , 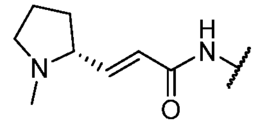 , 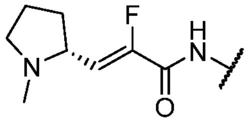 ,

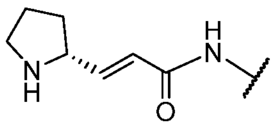 , 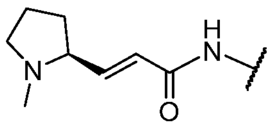 , 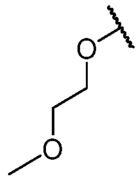 , 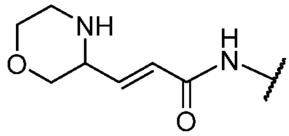 , or

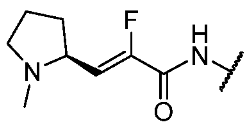 ;

n is 1 or 2;
G is N or C-CN.

**4.** A use of a compound having a structure of chemical formula 1, a pharmaceutically acceptable salt thereof, a solvate thereof, a solvate of the pharmaceutically acceptable salt thereof, or a crystal form thereof in the manufacture of a medicament for the treatment or prevention of a disease related to a ErbB2 mutant;

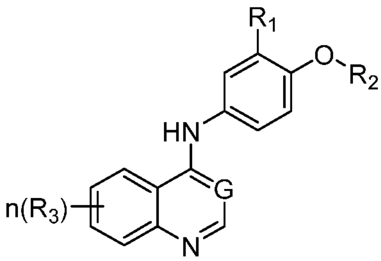 ;

Chemical formula 1

wherein $R_1$ is halogen or $C_{1-}C_6$ alkyl;
$R_2$ is

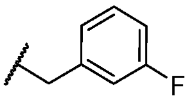 , 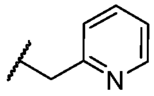 , 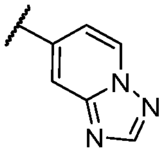 , 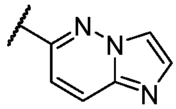 , 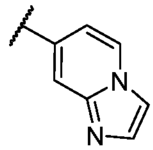 ,

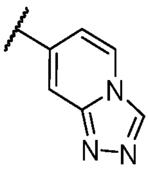 , 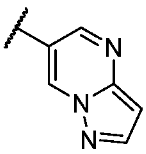 , 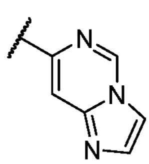 , or 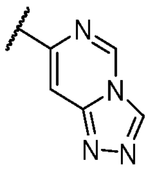 ;

each $R_3$ is independently

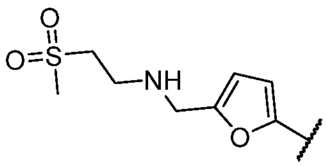 , 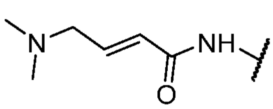 , 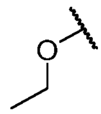 ,

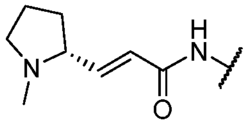 , 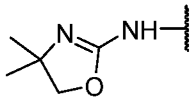 , 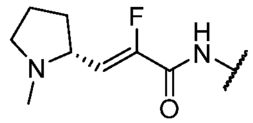 , 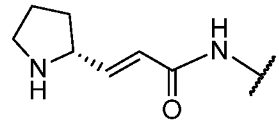 ,

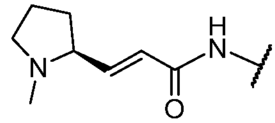 , 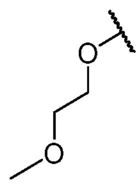 , 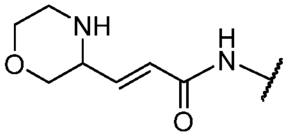 , or 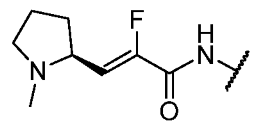 ;

n is 1 or 2;
G is N or C-CN.

**5.** The use according to claim 4, wherein in the compound having a structure of chemical formula 1, the pharmaceutically acceptable salt thereof, the solvate thereof, the solvate of the pharmaceutically acceptable salt thereof, or the crystal form thereof,

wherein $R_1$ is halogen or $C_1$-$C_6$ alkyl;
$R_2$ is

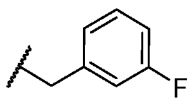 , 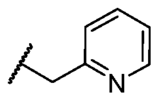 , or 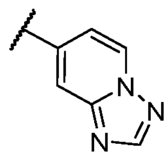 ;

each $R_3$ is independently

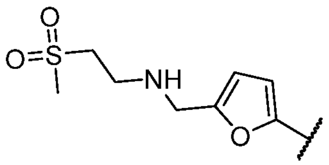, 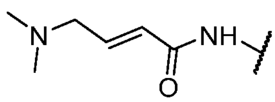, 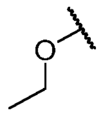,

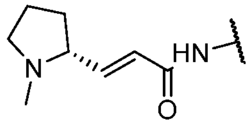, 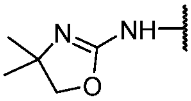, or 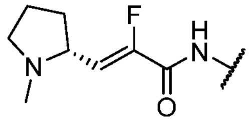;

n is 1 or 2;
G is N or C-CN.

**6.** The use according to claim 4, wherein in the compound having a structure of chemical formula 1, the pharmaceutically acceptable salt thereof, the solvate thereof, the solvate of the pharmaceutically acceptable salt thereof, or the crystal form thereof,

$R_1$ is halogen or $C_1$-$C_6$ alkyl;
$R_2$ is

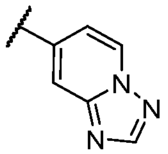, 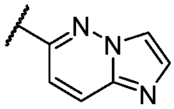, 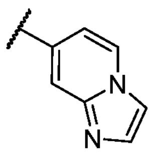, 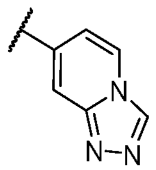, 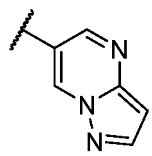,

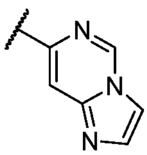, or 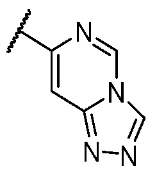;

each $R_3$ is independently

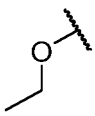, 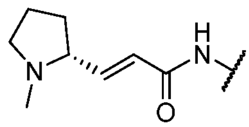, 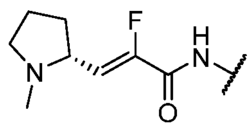,

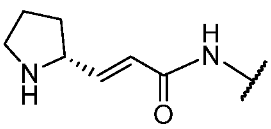, 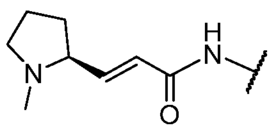, 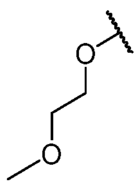, 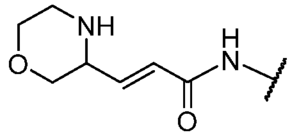,

or

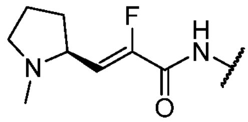;

n is 1 or 2;
G is N or C-CN.

**7.** The use according to claim 1 or 4, wherein in the chemical formula 1, when $R_1$ is halogen, the halogen is Cl; when $R_1$ is $C_{1-}C_6$ alkyl, the $C_{1-}C_6$ alkyl is methyl;
and/or,

when n is 1, $R_3$ is

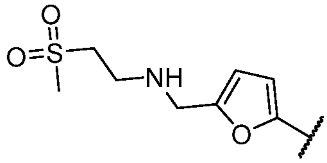, 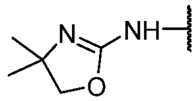, 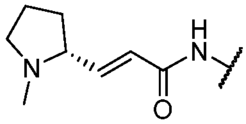,

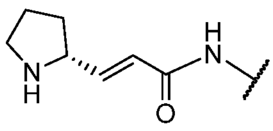, 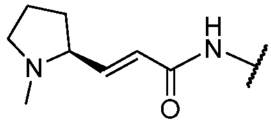, 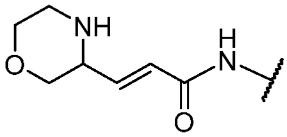, or 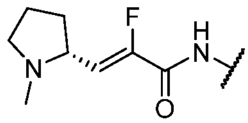,

preferably

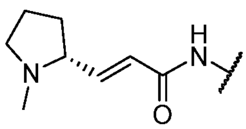, 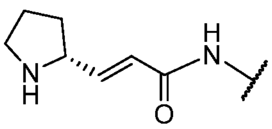, 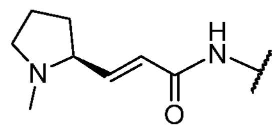,

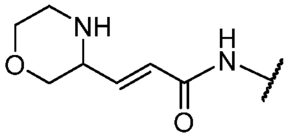, or 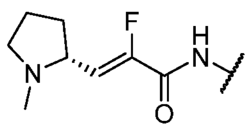;

when n is 2, $R_3$ is two neighboring groups, one of which is

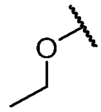 or 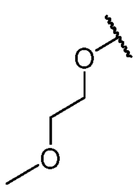,

and the other group is

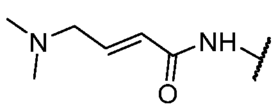, 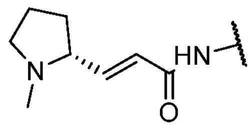, 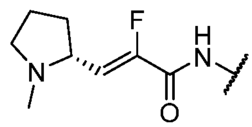,

or

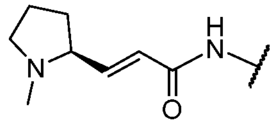,

preferably

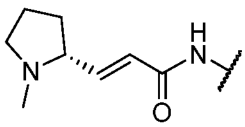, 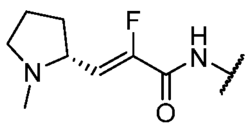,

or

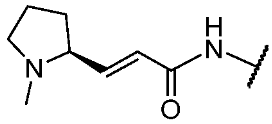;

and/or, $R_2$ is

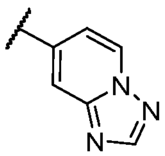, 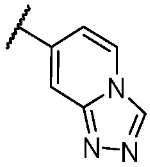, 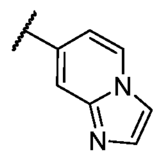, 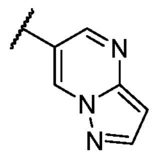, 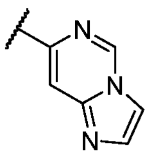,

or

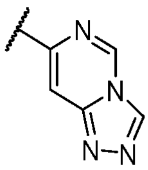;

preferably, the compound having a structure of chemical formula 1 is one or more compounds selected from (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide, Lapatinib, Tucatinib, Pyrotinib, Neratinib,

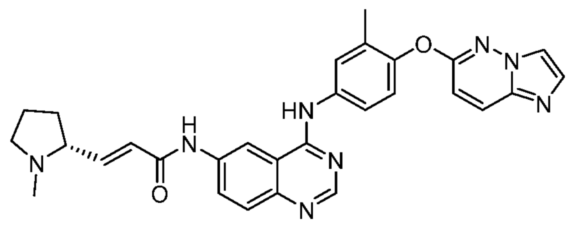

,

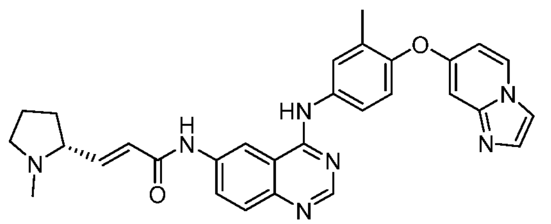

,

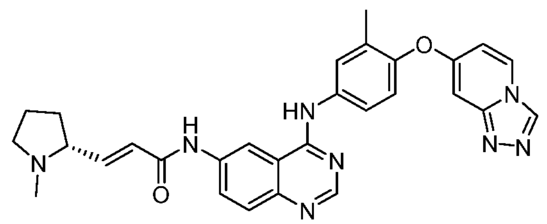

,

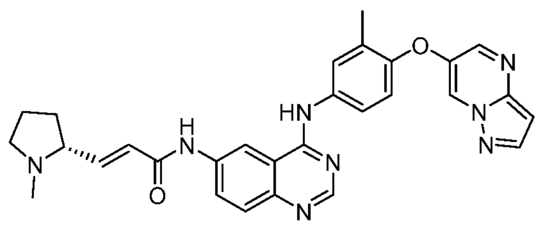

,

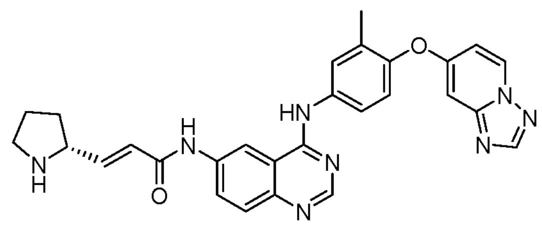

,

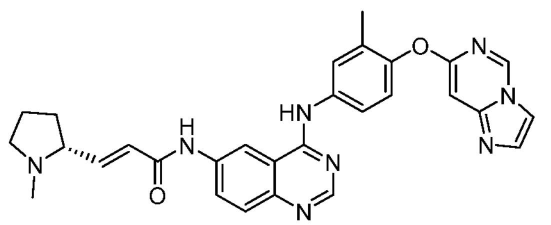

,

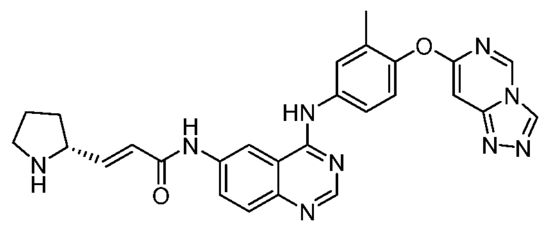

,

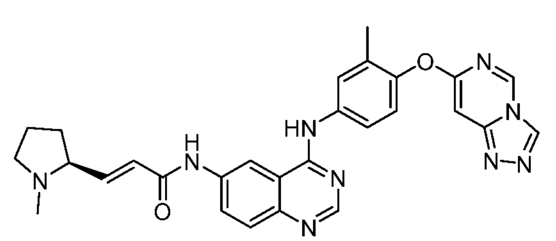

,

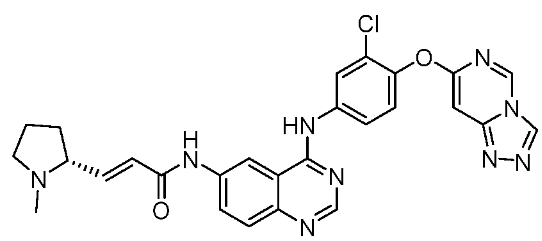

,

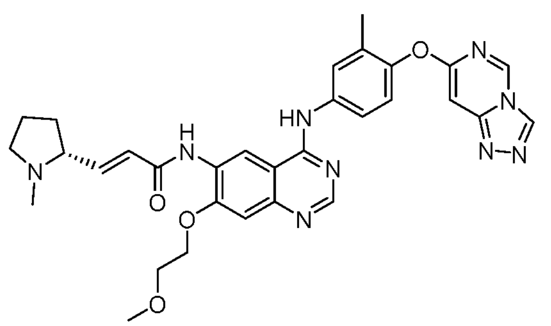

,

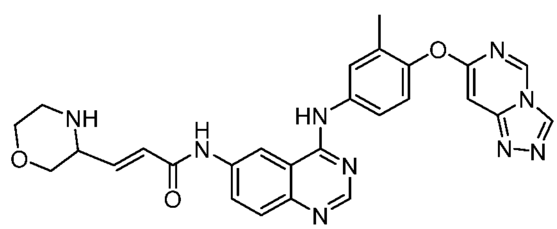

,

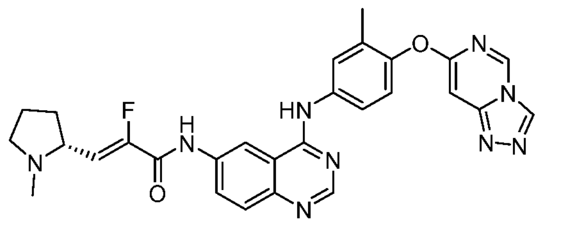
,
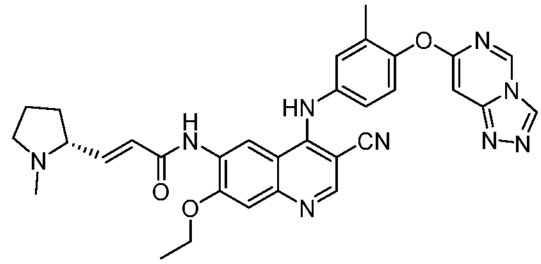
,

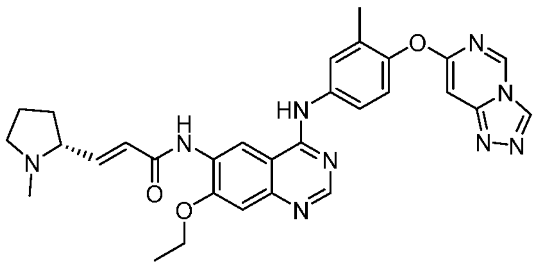
,
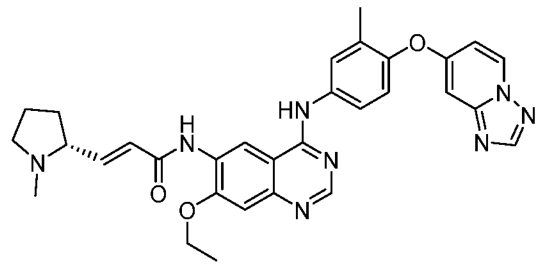
,

and

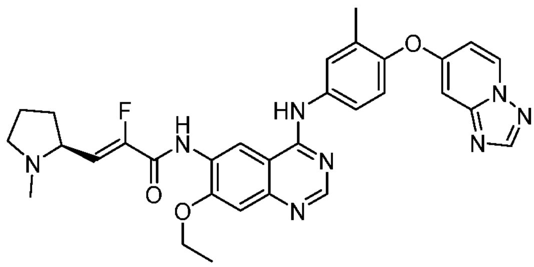
.

8. The use according to at least one of claims 1 to 7, wherein the ErbB2 mutant has a mutation type including one or more of D769H, D769Y, R896C, V777L, G766>VC, and A775_G776insYVMA.

9. The use according to claim 8, wherein the ErbB2 mutation type is D769H, D769Y, R896C, V777L, G766>VC, or A775_G776insYVMA, and the compound having a structure of chemical formula 1 is (R,Z)-N-(4-((4-([1,2,4]triazolo[1,5-a]pyridin-7-yloxy)-3-methylphenyl)amino)-7-ethoxyquinazolin-6-yl)-2-fluoro-3-(1-methylpyrrol-2-yl)acrylamide;

or, the ErbB2 mutation type is D769H, D769Y, R896C, V777L, or A775_G776insYVMA, and the compound having a structure of chemical formula 1 is Lapatinib;
or, the ErbB2 mutation type is D769H, D769Y, R896C, or V777L, and the compound having a structure of chemical formula 1 is Tucatinib;
or, the ErbB2 mutation type is D769H, D769Y, R896C, or V777L, and the compound having a structure of chemical formula 1 is Pyrotinib;
or, the ErbB2 mutation type is D769H, D769Y, R896C, or V777L, and the compound having a structure of chemical formula 1 is Neratinib;
or, the rbB2 mutation type is D769H, D769Y, R896C, or V777L, and the compound having a structure of chemical formula 1 is any one of the following structures:

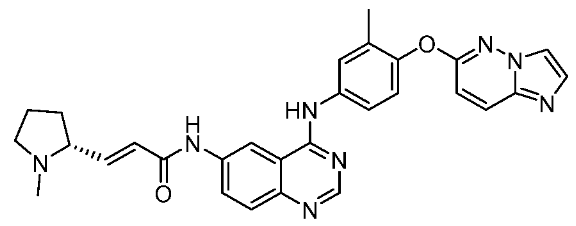,

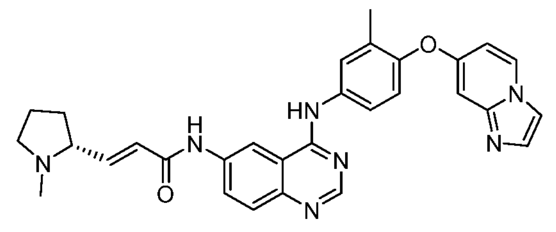,

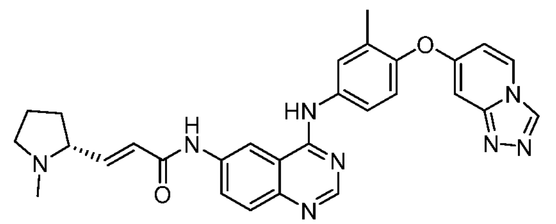,

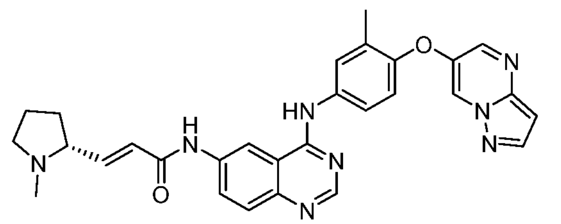,

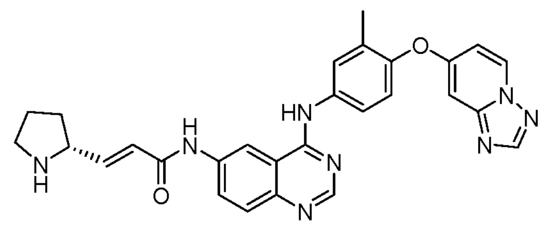,

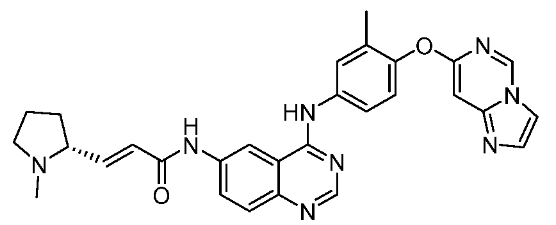,

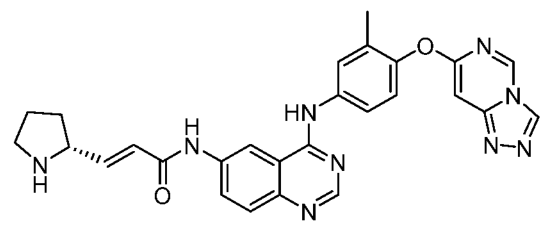,

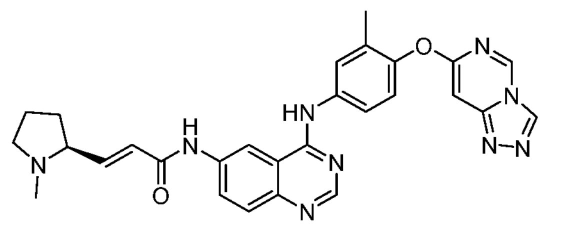,

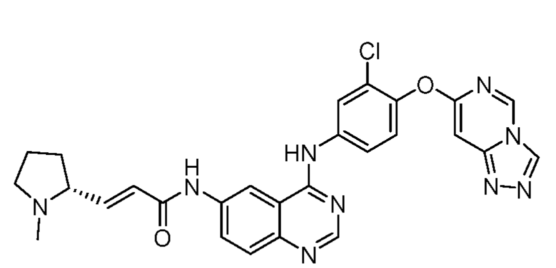,

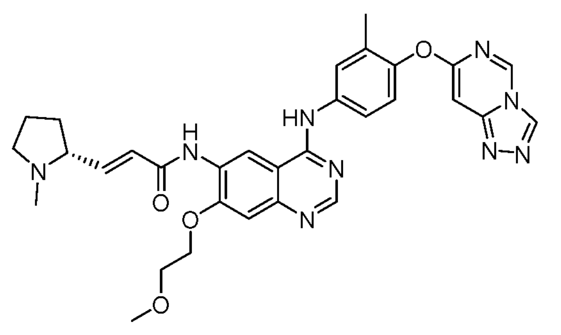,

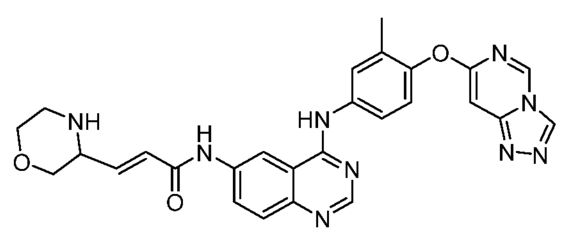,

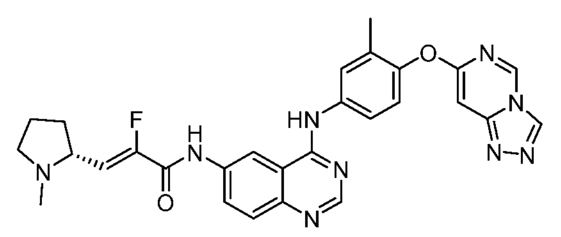,

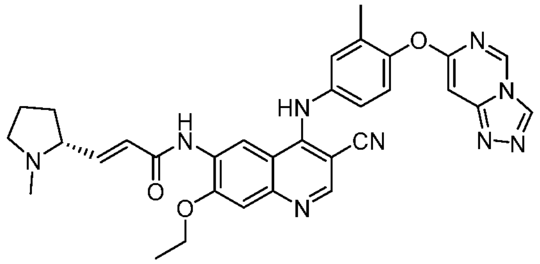

,

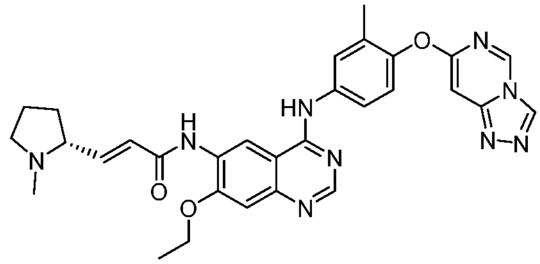

,

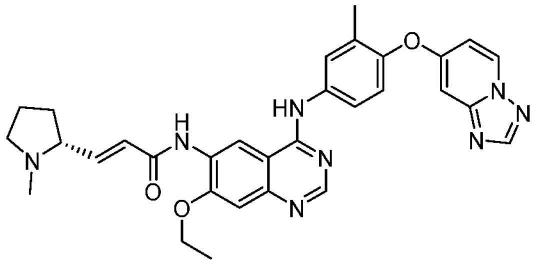

, or

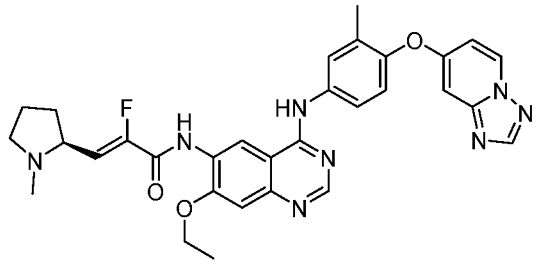

.

**10.** The use according to at least one of claims 4 to 6, wherein the disease related to the ErbB2 mutant is ErbB2 mutant cancer;

the ErbB2 mutant cancer preferably comprises breast cancer, colon cancer, head and neck cancer, bladder cancer, gastric cancer, ovarian cancer, liver cancer, or lung cancer;
the lung cancer is further preferably non-small cell lung cancer.

**11.** The use according to at least one of claims 4 to 6, wherein the treatment is inhibition of tumor metastasis and/or growth.

**12.** The use according to at least one of claims 1 to 11, wherein the medicament further comprises a pharmaceutically acceptable carrier.

**13.** The use according to at least one of claims 1 to 11, wherein the compound having a structure of chemical formula 1 is the sole active ingredient of the medicament, or act as an active ingredient in conjunction with other ErbB2 mutant inhibitors or medicaments for the treatment of cancer.

**14.** The use according to at least one of claims 1 to 11, wherein the content of the compound having a structure of chemical formula 1 is the content that achieves a therapeutically effective amount.

**INTERNATIONAL SEARCH REPORT**

International application No. **PCT/CN2022/084650**

**A. CLASSIFICATION OF SUBJECT MATTER**

A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

DWPI; SIPOABS; CNKI; CNABS; STNext; Web of Science: 上海医药集团股份有限公司, ErbB2, 突变, 拉帕替尼, 妥卡替尼, 吡咯替尼, 来那替尼, Mutation, Lapatinib, Tucatinib, Pyrotinib, Neratinib, D769H, D769Y, R896C, V777L, G766>VC, A775_G776insYVMA

**C. DOCUMENTS CONSIDERED TO BE RELEVANT**

| Category* | Citation of document, with indication, where appropriate, of the relevant passages | Relevant to claim No. |
|---|---|---|
| X | WO 2007095038 A2 (NOVARTIS AG. et al.) 23 August 2007 (2007-08-23) abstract, and claims 1-11 | 1-14 |
| Y | WO 2019042409 A1 (SHANGHAI PHARMACEUTICALS HOLDING CO., LTD.) 07 March 2019 (2019-03-07) abstract, and claims 1-29 | 1-14 |
| X | NAGANO, M. et al. "High-Throughput Functional Evaluation of Variants of Unknown Significance in ERBB2." *Clinical Cancer Research,* Vol. 24, No. 20, 15 October 2018 (2018-10-15), pp. 5112-5122 | 1-14 |
| Y | NAGANO, M. et al. "High-Throughput Functional Evaluation of Variants of Unknown Significance in ERBB2." *Clinical Cancer Research,* Vol. 24, No. 20, 15 October 2018 (2018-10-15), pp. 5112-5122 | 1-14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 June 2022** | **27 June 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

**INTERNATIONAL SEARCH REPORT**

International application No.
**PCT/CN2022/084650**

**C. DOCUMENTS CONSIDERED TO BE RELEVANT**

| Category* | Citation of document, with indication, where appropriate, of the relevant passages | Relevant to claim No. |
|---|---|---|
| X | SHIMAMURA, T. et al. "Non-small-cell lung cancer and Ba/F3 transformed cells harboring the ERBB2 G776insV_G/C mutation are sensitive to the dual-specific epidermal growth factor receptor and ERBB2 inhibitor HKI-272." *Cancer Research,* Vol. 66, No. 13, 01 July 2006 (2006-07-01), pp. 6487-6491 | 1-14 |
| X | WU, Yanyan et al. "Successful treatment of pyrotinib for bone marrow metastasis induced pancytopenia in a patient with non-small-cell lung cancer and ERBB2 mutation." *Thorac Cancer,* Vol. 11, No. 7, 26 May 2020 (2020-05-26), pp. 2051-2055 | 1-14 |
| X | MINAMI, Y. et al. "The major lung cancer-derived mutants of ERBB2 are oncogenic and are associated with sensitivity to the irreversible EGFR/ERBB2 inhibitor HKI-272." *Oncogene,* Vol. 26, No. 34, 26 July 2007 (2007-07-26), pp. 5023-5027 | 1-14 |
| A | WO 2020205521 A1 (BOARD OF REGENTS, THE UNIVERSTIY OF TEXAS SYSTEM) 08 October 2020 (2020-10-08) description abstract, claims 1-123 | 1-14 |

Form PCT/ISA/210 (second sheet) (January 2015)

**INTERNATIONAL SEARCH REPORT**
**Information on patent family members**

International application No.
**PCT/CN2022/084650**

| Patent document cited in search report | | | Publication date (day/month/year) | Patent family member(s) | | | Publication date (day/month/year) |
|---|---|---|---|---|---|---|---|
| WO | 2007095038 | A2 | 23 August 2007 | None | | | |
| WO | 2019042409 | A1 | 07 March 2019 | JP | 2020531553 | A | 05 November 2020 |
| | | | | CN | 109422755 | A | 05 March 2019 |
| | | | | EP | 3677583 | A1 | 08 July 2020 |
| | | | | US | 2020190091 | A1 | 18 June 2020 |
| WO | 2020205521 | A1 | 08 October 2020 | EP | 3946293 | A1 | 09 February 2022 |
| | | | | KR | 20210149103 | A | 08 December 2021 |
| | | | | AU | 2020254499 | A1 | 28 October 2021 |
| | | | | US | 2022143023 | A1 | 12 May 2022 |
| | | | | IL | 286742 | D0 | 31 October 2021 |
| | | | | CN | 113939284 | A | 14 January 2022 |
| | | | | SG | 11202110669 W | A | 28 October 2021 |
| | | | | CA | 3131864 | A1 | 08 October 2020 |
| | | | | BR | 112021019489 | A2 | 08 February 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021103581923
- CN 109422755 A
- WO 2019042409 A1
- WO 2017148391 A1


### Non-patent literature cited in the description


- *Nature,* 2018, vol. 554 (7691), 189-194
- *Oncotarget,* 2018, vol. 9 (11), 9741-9750
- *J Clin Oncol,* 2020
- *BMC Cancer,* 2016, vol. 16, 828
- *Cancer,* 30 August 2019